(19) Europäisches Patentamt
European Patent Office
Office européen des brevets

(11) EP 3 995 575 A1

(12) **EUROPEAN PATENT APPLICATION**
published in accordance with Art. 153(4) EPC

(43) Date of publication:
**11.05.2022 Bulletin 2022/19**

(21) Application number: **20837838.0**

(22) Date of filing: **03.07.2020**

(51) International Patent Classification (IPC):
***C12N 15/10*** *(2006.01)* ***C12N 15/115*** *(2010.01)*
***C12Q 1/68*** *(2018.01)* ***G01N 33/543*** *(2006.01)*

(86) International application number:
**PCT/KR2020/008759**

(87) International publication number:
**WO 2021/006570 (14.01.2021 Gazette 2021/02)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **05.07.2019 KR 20190081193**

(71) Applicant: **SB Bioscience Co., Ltd.**
**Buk-gu, Gwangju 61005 (KR)**

(72) Inventors:
- **KIM, Min Gon**
  **Gwangju 61005 (KR)**
- **KANG, Ju Young**
  **Gwangju 61005 (KR)**

(74) Representative: **Botti, Mario**
**Botti & Ferrari S.p.A.**
**Via Cappellini, 11**
**20124 Milano (IT)**

# (54) APTAMER SELECTION METHOD AND IMMUNITY ANALYSIS METHOD USING APTAMER

(57) The present invention relates to an aptamer screening method, and the aptamer screened by the screening method of the present invention binds to a site other than a site where the antibody binds to the target substance to be applicable in various fields such as sandwich-type biosensors and reduce a significant time without requiring a separate pairing selection process. In addition, such an aptamer has excellent stability and sensitivity compared to conventional preparations comprising an antibody, can be mass-produced at low cost in a short time by a chemical synthesis method, and is easily transformed in various ways to increase a binding force. In addition, the immunoassay method using the aptamer screened by the aptamer screening method of the present invention selectively amplifies only the aptamer binding to the target substance in a heterogeneous sandwich structure to detect a relative fluorescence signal, thereby detecting the target substance sensitively and quickly.

[FIG. 2]

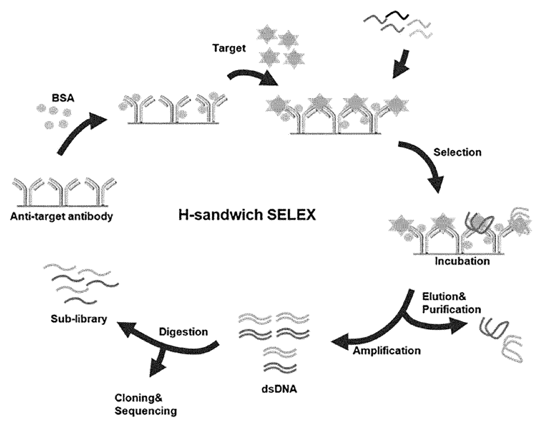


EP 3 995 575 A1

## Description

[Technical Field]

The present invention relates to a screening method of an aptamer and an immunoassay method using the aptamer, and more particularly, to a heterogeneous sandwich type immunoassay method using a nucleic acid amplification technique.

[Background Art]

An aptamer is a nucleic acid molecule having high specific binding affinity with molecules through interactions other than the formation of classical Watson-Crick base pair. The aptamer has a characteristic of being able to specifically bind to a selected target, and for example, may determine the presence or absence of the target and inhibit or activate a function of the target through aptamer binding. The aptamer capable of specifically binding to proteins including a growth factor, a transcription factor, an enzyme, immunoglobulin and a receptor have been generated from a random sequence oligonucleotide library by an in vitro screening process.

As a result of a series of structural studies, it has been shown that the aptamer may use the same types of binding interactions (e.g., hydrogen bond, electrostatic complementarity, hydrophobic contact, and steric exclusion) that impart high affinity and selective binding in an antibody-antigen complex. Since the aptamer has high selectivity and affinity, biological efficacy, and excellent pharmacokinetic properties, it is very easily applicable not only to a field of diagnosis but also to a field of therapeutic agents using the same.

As a screening method for the aptamer, a 'Systematic Evolution of Ligands by Exponential Enrichment (SELEX)' method has been widely used. The SELEX method is a method for in vitro development of a nucleic acid molecule having high specific binding to a target molecule, which is disclosed in U.S. Patent Application No. 07/536,428 (now abandoned) filed on June 11, 1990, U.S. Patent No. 5,475,096 (title of invention: "Nucleic Acid Ligands"), U.S. Patent No. 5,270,163 (title of invention: "Nucleic Acid Ligands"), and the like.

As a basic principle of the SELEX method, nucleic acids are sufficient to form various 2D and 3D structures, sufficient to specifically bind to substantially any chemical compound (regardless of monomers or polymers), and have chemical versatility usable in monomers. Specifically, in the case of the SELEX method, generally, oligonucleotides which do not bind to the target substance are removed from used oligonucleotide aggregates using an affinity chromatography method, and a conjugate of the oligonucleotide and the target substance may be selectively obtained. As described above, it is possible to discover an oligonucleotide that specifically binds to the target substance, that is, an aptamer specific to the target substance by separating and amplifying the oligonucleotide from the target substance in the selected conjugate of the oligonucleotide and the target substance. However, in a conventional SELEX method, a process of immobilizing the target substance on a chromatography column resin was required for binding of the target substance and the oligonucleotide and the selective separation of the conjugate. Therefore, there are disadvantages that it is very difficult to discover specific aptamers to organic/inorganic molecules that do not contain functional groups for easy chemical reaction, and a process of discovering aptamers for proteins or some organic molecules having various functional groups in a single molecule is also somewhat complex.

A new type of immunoassay method for the diagnosis of known infectious diseases by immuno-PCR integrates PCR with an antibody conjugated with a DNA probe for highly sensitive detection of proteins. After binding of a protein target and an antibody pair forms a sandwich-type immune complex, a DNA probe linked to the detection antibody is amplified, resulting in higher sensitive detection than being achieved with conventional immunoassays.

However, this immunoassay method still has several disadvantages associated with the use of paired antibodies. First, an antibody production process does not include an additional selection step to reduce the interaction between the two antibodies. Accordingly, a low signal-to-noise ratio may limit the sensitivity enhancement promoted by probe amplification. Moreover, this method still requires antibody screening to select optimal paired antibodies, which slows down the development of new immunoassays. Finally, the synthesis of the detection antibody conjugated with the DNA probe may induce antibody degradation due to the use of many chemical reagents and requires a complex step including amino modifications to the DNA probe.

[Prior Arts]

(Non-Patent Document 0001) Fischer, Nicholas O et al. "Protein detection via direct enzymatic amplification of short DNA aptamers." Analytical biochemistry vol. 373,1 (2008): 121-8.

[Disclosure]

[Technical Problem]

An object of the present invention is to provide a screening method of an aptamer capable of specifically binding to a site of a target substance other than an antibody binding site. Another object of the present invention is to provide a heterogeneous sandwich-type immunoassay method using an aptamer and a nucleic acid amplification technique.

[Technical Solution]

An exemplary embodiment of the present invention provides a screening method of an aptamer.

The screening method of the present invention includes an antibody immobilization step of immobilizing a specific antibody to a target substance to a support; a first reaction step of adding and reacting the target substance to the antibody-immobilized support; a second reaction step of adding and reacting an aptamer library to the support where the first reaction step is completed; and a first elution step of eluting the aptamer binding to the target substance in the second reaction step.

The screening method of the present invention may further include an amplification step of amplifying a nucleic acid of the aptamer eluted in the first elution step; a second-1 reaction step of adding and reacting the amplified nucleic acid of the aptamer into the support on which the first reaction step has been completed; and a second elution step of eluting the aptamer binding to the target substance in the second-1 reaction step.

The "aptamer" of the present invention refers to a single-stranded nucleic acid chain adopting a specific tertiary structure binding to a molecular target with high specificity and affinity, which is equal to a single clone antibody through an interaction other than the conventional Watson-Crick base pairing. The aptamer may be a nucleic acid, DNA or RNA, preferably a single-stranded DNA, but is not limited thereto. When the aptamer of the present invention is RNA, the aptamer may be produced by transcribing a DNA library in vitro using T7 RNA polymerase or modified T7 RNA polymerase.

The "nucleic acid" used herein refers to any type of nucleic acid, such as DNA and RNA, and variants thereof, such as peptide nucleic acid (PNA), locked nucleic acid (LNA), and combinations thereof, modifications thereof, such as modified nucleotides, and the like. The terms "nucleic acid" and "oligonucleotide" and "polynucleotide" are used interchangeably. The nucleic acids may be prepared using a recombinant expression system and, optionally, purified from other purified and chemically synthesized natural sources and the like. In the case of a chemically synthesized molecule, the nucleic acid may include a nucleoside analogue, such as an analogue having a chemically modified base or sugar, a modification of the backbone, and the like. The nucleotide sequence of the nucleic acid is indicated in a 5'-3' direction unless otherwise indicated.

The "aptamer library" used herein includes a plurality of single-stranded DNAs (ssDNAs) or RNAs having the ability to bind to a target substance, and the aptamer library may include at least one single-stranded DNA nucleotide sequence or RNA nucleotide sequence selected from the group consisting of different nucleotide sequences.

The aptamer of the present invention may include a chemically modified form. In general, wild-type nucleic acid molecules that do not have chemical modifications are susceptible to degradation by nucleases. Accordingly, the nucleic acid aptamer of the present invention may introduce any type of chemical modification that imparts resistance to various nucleases known in the art. For example, the chemical modification includes 2'-amino pyrimidine, 2'-fluoro pyrimidine, 2'-O-methyl ribose purine, pyrimidine, and the like, and may be performed by a method of modifying a phosphodiester bond of a backbone with a phosphorothioate bond. In addition, by linking a 3'-terminus to 3'-3', the 3'-terminus may be capped to increase resistance to nucleases, and polyethyleneglycol (PEG) is attached as a polymer to reduce a renal filtration renal filtration rate.

The single-stranded nucleotide sequence of the present invention consists of a forward or reverse primer nucleotide sequence for amplification at both terminals, and the center of the primer nucleotide sequence may consist of 30 to 50 nucleotide sequences, preferably 35 to 45 nucleotide sequences, and more preferably 40 nucleotide sequences, but is not limited thereto. When the nucleotide sequences at the center are less than 30 and more than 50, there may be no ability of specifically binding to the target substance or the single-stranded nucleic acid may not sufficiently form a secondary or tertiary structure.

In one embodiment of the present invention, the aptamer may consist of a nucleotide sequence represented by SEQ ID NO: 2 below. In the nucleotide sequence of SEQ ID NO: 2 below, N is an integer of 40 nucleotide sequences, and the nucleotides are independently selected from the group consisting of A (adenine), C (cytosine), G (guanine) and T (thymine). In this case, when the aptamer is RNA, U (uracil) is used instead of T.

5'-ATC CAG AGT GAC GCA GCA -[NNNNNNNNNNNNNNNNNNNNNNNNNNNNNNNNNNNNNNNN]- TG GAC ACG GTG GCT TAG T-3' (SEQ ID NO: 2)

Hereinafter, each step will be described in detail with reference to FIG. 2.

**Antibody immobilization step;**

In the antibody immobilization step of the present invention, an antibody specific to a target substance is immobilized to a support.

The antibody immobilization step of the present invention is to immobilize the antibody specific to the target substance to the support, and through this process, an aptamer capable of binding to a site other than an antibody-specific binding site of the target substance may be specifically selected.

In the antibody immobilization step of the present invention, a portion excluding an antigen-binding site to the antibody capable of binding to the target substance is uniformly and stably bound to the surface of the support, and ionic bond, covalent bond, van der Waals bond, or plasma grafting method, or combinations thereof may be used to be suitable for a type of support thereof according to a general method.

The "specific antibody to the target substance" of the present invention is a substance specifically binding to the target substance to cause an antigen-antibody reaction, and may include all of immunoglobulin G (IgG), IgA, IgM, IgD and IgE antibodies so long as the antibody may cause an antigen-antibody reaction with the target substance to be targeted of the present invention. The basic structure of the antibody is a Y-shaped protein, and includes variable sites having specific amino acid sequences capable of binding to an antigen in upper two branches of the Y-shape, and includes a constant site responsible for structural stability and the like.

The "support" of the present invention is a base on which the antibody may be immobilized, and may include any substance which may bind to an amino acid site other than the variable sites of the antibody, and for example, may be at least one selected from the group consisting of a nitrocellulose membrane; a polyvinilidenfluoride (PVDF) membrane; a well plate synthesized with polyvinyl resin or polystyrene resin; and slide glass, preferably a well plate, but is not limited thereto.

The well plate of the present invention may consist of 6-wells, 12-wells, 24-wells, 48-wells or 96-wells, and preferably a 96-well plate, but is not limited thereto.

**First reaction step;**

In the first reaction step of the present invention, the target substance is added and reacted to the support to which the antibody is immobilized.

The first reaction step of the present invention is a step for binding the target substance to the antibody through the antigen-antibody reaction, and through this process, an aptamer capable of binding to a site other than the antibody-specific binding site of the target substance may be specifically screened.

The "target substance" of the present invention refers to a substance to which an aptamer specifically binds, and may includes all substances as long as an antigen-antibody reaction can occur, and for example, may be at least one selected from the group consisting of metal ions, compounds, nucleic acids, proteins, peptides and cells, and may preferably be proteins, but is not limited thereto.

In the first reaction step of the present invention, before the process of reacting the target substance with the antibody (after the immobilization step), a step of adding and reacting a blocking buffer may be further performed. As such, when the immobilized antibody reacts with the blocking substance after the immobilization step, the efficiency of screening the aptamer that specifically binds only to the target substance without binding to the antibody may be remarkably increased.

The "blocking buffer" of the present invention is a buffer containing a blocking substance to prevent a non-specific reaction occurring when the aptamer binds to the antibody, and the blocking substance may include all substances for preventing the non-specific reaction in which the aptamer binds to the antibody, and for example, non-fat-milk or bovine serum albumin (BSA), but is not limited thereto.

**Recovery step;**

After the first reaction step of the present invention, the method may further include a step of adding and reacting the aptamer library to the support immobilized with the antibody; and a recovery step of recovering a supernatant containing the aptamer library that has not reacted with the antibody.

When the recovery step of the present invention is additionally performed, there is an advantage that noise that may be generated in the screening process of the aptamer may be very effectively removed by selectively removing the aptamer that interacts with the antibody other than the target substance.

**Second reaction step;**

In the second reaction step of the present invention, the aptamer library is added and reacted to the support on which the first reaction step is completed.

The second reaction step of the present invention is a step of allowing the aptamer to bind to the target substance, and through this process, an aptamer that specifically binds to the target substance may be screened.

The "specific binding" of the present invention means a non-covalent physical bond between the aptamer of the present invention and a nuclear protein of its target substance. Upon binding between the aptamer of the present invention and the target substance, a dissociation constant (Kd) value may be 0.3 μM to 5 μM, preferably 0.5 μM to 4 μM, but is not limited thereto. In the case of a dissociation constant of less than 0.5 μM or more than 5 μM, it may not be easy to detect the target substance.

**First elution step**

In the second reaction step of the present invention, the aptamer specifically binding to the target substance is eluted.

The first elution step of the present invention corresponds to a step of separating the binding between the target substance and the aptamer in order to obtain only the aptamers binding to the target substance in the aptamer library.

The elution step of the present invention may be all general methods for separating the binding between the target substance and the aptamer, and may be performed by for example, a centrifugation method or a method of reacting with an acid or base solution, but the present invention is not limited thereto.

**Amplification step; Second-1 reaction step; and Second elution step;**

The screening method of the aptamer the present invention may further include an amplification step of amplifying a nucleic acid of the aptamer eluted in the first elution step; a second-1 reaction step of adding and reacting the amplified nucleic acid of the aptamer into the support on which the first reaction step has been completed; and a second elution step of eluting the aptamer binding to the target substance in the second-1 reaction step.

The amplification step; the second-1 reaction step; and the second elution step of the present invention are steps for performing a specific reaction between the target substance and the aptamer multiple times, and finally, the specificity of the screened aptamer may be increased.

The "amplification step" of the present invention may use any method for amplifying a nucleic acid of the aptamer, and for example, may be performed through a polymerase chain reaction, but is not limited thereto.

The amplification step; the second-1 reaction step; and the second elution step of the present invention may be repeated 2 to 30 times in sequence, preferably repeated 5 to 15 times, and more preferably repeated 10 times, but is not limited thereto. When the repetition step is less than 2 times, the specificity of a desired aptamer cannot be sufficiently increased, and when the repetition step is more than 30 times, excessive cost and time may be required.

In the aptamer screened in the screening method of the present invention, a detectable label may bind to a 3' terminus or 5' terminus of the aptamer. Such a detectable label may be a moiety that may be detected by a detection method known in the art, and may bind to a specific base and a specific structure of the aptamer according to a general method.

The detectable label of the present invention may be, for example, at least one label selected from the group consisting of an optical label, an electrochemical label, and a radioisotope, but is not limited thereto.

The optical label of the present invention may be a fluorescent substance or an enzyme.

The fluorescent substance of the present invention may be at least one selected from the group consisting of fluorescein, biotin, 6-FAM, rhodamine, Texas Red, tetramethylrhodamine, carboxyrhodamine, carboxyrhodamine 6G, carboxyrodol, carboxyrhodamine 110, Cascade Blue, Cascade Yellow, coumarin, Cy2 (cyanine 2), Cy3 (cyanine 3), Cy3.5 (cyanine 3.5), Cy5 (cyanine 5), Cy5.5 (cyanine 5.5), Cy-chromium, phycoerythrin, peridinine chlorophyll-a protein (PerCP), PerCP-Cy5.5, 6-carboxy-4',5'-dichloro-2',7'-dimethoxyfluorescein (JOE), NED, 5-(and-6)-carboxy-X-rhodamine (ROX), HEX, Lucifer Yellow, Marina Blue, Oregon Green 488, Oregon Green 500, Oregon Green 514 , Alexa Fluor, 7-amino-4-methylcomarin-3-acetic acid, Bodipy FL, Bodipy FL-Br 2, Bodipy 530/550, and conjugates thereof, but is not limited thereto. The enzyme of the present invention may be an enzyme used in enzyme-linked immunosorbent assay (ELISA), and for example, alkaline phosphatase, horseradish peroxidase, luciferase, or glucose oxidase. When the enzyme is used as the optical label, in order to induce a chemiluminescent reaction, preferably, a chemiluminescent substance may include luminol, streptividin, isoluminol, luciferin, lucigenin, 3-(2'-spiroadamantane)-4-methoxy-4-(3"-phosphoryloxy)phenyl-1,2-dioxetane (AMPPD), disodium 3-(4-methoxyspiro {1,2-dioxetane-3,2'-(5'-chloro)tricyclo[3.3.1.13,7]decan}-4-yl) phenyl phosphate (CSPD), etc., but is not limited thereto.

The optical label of the present invention includes a fluorescence donor chromophore and a fluorescence acceptor chromophore separated by an appropriate distance, and may be a fluorescence resonance energy transfer (FRET) pair in which the fluorescence of the donor is suppressed by the acceptor. The donor chromophore may include FAM, TAMRA, VIC, JOE, Cy3, Cy5 and Texas Red, preferably FAM, but is not limited thereto.

The radioactive isotope of the present invention may be, for example, at least one selected from the group consisting of 1241, 1251, 111In, 99mTc, 32P and 35S, but is not limited thereto.

According to another embodiment of the present invention, there is provided an aptamer screened by the aptamer screening method.

According to yet another embodiment of the present invention, there is provided an immunoassay kit including the aptamer screened by the aptamer screening method. The immunoassay kit may further include an antibody used in the aptamer screening method as well as the aptamer screened by the aptamer screening method.

According to yet another embodiment of the present invention, there is provided an immunoassay method using the aptamer screened by the aptamer screening method.

According to yet another embodiment of the present invention, there is provided an immunoassay method using the aptamer, and the immunoassay method may include mixing a detection sample with an aptamer specifically binding to a target substance to be detected; reacting the mixture with a binding substance immobilized to the support and specifically binding to the target substance to form a complex of aptamer-target substance-binding substance; and amplifying the aptamer.

The aptamer is preferably an aptamer screened by the aptamer screening method disclosed herein.

Specifically, the immunoassay method using the aptamer uses the aptamer screened by the aptamer screening method. The immunoassay method using the aptamer screened by the aptamer screening method may include mixing a detection sample with the aptamer screened by the aptamer screening method which specifically binds to the target substance to be detected; reacting the mixture with a binding substance immobilized to the support and specifically binding to the target substance to form a complex of aptamer-target substance-binding substance; and amplifying the aptamer.

The aptamer screening method may include a binding substance immobilization step of immobilizing a binding substance specific to a target substance to a support; a first reaction step of adding and reacting the target substance to the binding substance-immobilized support; a second reaction step of adding and reacting an aptamer library to the support where the first reaction step is completed; and a first elution step of eluting the aptamer binding to the target substance in the second reaction step.

The aptamer screening method may further include adding a blocking buffer and reacting the blocking substance with the binding substance before the immobilization step. This is to prevent a non-specific reaction caused by binding of the aptamer and the binding substance when performing the immunoassay method, and by the blocking step, the aptamer that specifically binds to the target substance may be efficiently screened.

The aptamer screening method of the present invention may further include an amplification step of amplifying a nucleic acid of the aptamer eluted in the first elution step; a second-1 reaction step of adding and reacting the amplified nucleic acid of the aptamer into the support on which the first reaction step has been completed; and a second elution step of eluting the aptamer binding to the target substance in the second-1 reaction step.

In addition, after the second reaction step, the aptamer screening method of the present invention may further include a recovery step of recovering the supernatant containing the aptamer library that does not react with the binding substance.

The amplification step; the second-1 reaction step; and the second elution step may be repeated 2 to 30 times, 2 to 20 times, or 1 to 10 times in sequence.

The present inventors provides a new disease diagnosis system based on recombinase polymerase amplification (RPA) using a heterogeneous sandwich (H-sandwich) DNA aptamer, and the method was named H-sandwich RPA.

The DNA aptamer used as the detection probe of the target substance may be screened by the aptamer screening method of the present invention without requiring an additional pair-screening step, and the aptamer binds to another site of a target substance to be detected, for example, an antigen binding to an H-sandwich binding substance, for example, an antibody. In addition, it is preferable that the aptamer specifically binds only to the target substance to be detected without interacting with the binding substance, for example, without binding.

In the complex of the aptamer-target substance-binding substance, the aptamer binding to the target substance is amplified into double-stranded DNA by an amplification reaction, and binds to an intercalating dye to generate a fluorescence signal at a specific wavelength. The intensity of the generated fluorescence signal according to an amount of amplified DNA shows a difference, and the presence or absence of an antigen may be determined through a relative fluorescence intensity value.

In addition, since only a DNA aptamer may be amplified using two different bioreceptors, the DNA aptamer has high sensitivity and specificity, and the presence or absence of the target substance in real human samples may be easily determined without a matrix effect.

Therefore, the application of the H-sandwich RPA for detecting disease biomarkers present in trace amounts in the body may be utilized as a simple and ultra-sensitive biosensor and an analysis tool.

A specific embodiment (H-sandwich RPA) of the immunoassay method of the present invention is schematically illustrated in FIG. 6.

A target substance (e.g., antigen) and an aptamer specific to the target substance are pre-incubated and then react with a binding substance (e.g., capture antibody) immobilized in wells. In the presence of the target substance, a complex (e.g., immune complex) of a heterogeneous sandwich structure consisting of an aptamer-target substance-binding substance (aptamer-antigen-antibody) is formed.

In order to induce a DNA aptamer amplification reaction (e.g., RPA reaction) and detect the generated fluorescence signal, a reaction solution mixture is prepared with a primer, a reagent and an intercalating dye at appropriate concentrations and then subjected to isothermal reaction in wells at 37°C for 20 minutes.

The dsDNA generated through the DNA aptamer amplification reaction binds to an intercalating dye at a specific wavelength to induce a fluorescence signal, and as a result, a difference in fluorescence signal occurs depending on the amount of the aptamer binding to the target substance and the presence of the target substance in the sample may be detected.

The "sample" is not particularly limited so long as containing a sample (target substance) to be detected. Illustratively, the sample may be a biological sample, for example, a biological fluid or a biological tissue. Examples of the biological fluid may include urine, blood (whole blood), plasma, serum, saliva, semen, stool, sputum, cerebrospinal fluid, tear, mucus, amniotic fluid, etc. The biological tissue is a cluster of cells, and may correspond to connective tissue, epithelial tissue, muscular tissue, neural tissue, etc., as a specific type of set with intracellular substances which typically form one of structural substances of human, animal, plant, bacteria, fungal or viral structures. In addition, examples of the biological tissue may also include organs, tumors, lymph nodes, arteries, and individual cell(s).

The binding substance may be selected from the group consisting of antibodies, antigens, nucleic acids, aptamers, hapten, antigen proteins, DNA, RNA binding proteins, cationic polymers, and mixtures thereof, and may use any substance which binds specifically to a target substance to be detected.

The cationic polymer may be selected from the group consisting of chitosan, glycol chitosan, protamine, polylysine, polyarginine, polyamidoamine (PAMAM), polyethylenimine, dextran, hyaluronic acid, albumin, polymer polyethyleneimine (PEI), polyamine, polyvinyl amine (PVAm), and mixtures thereof.

As the binding substance, it is preferable to use a binding substance (e.g., antibody) used in the aptamer selection method. That is, it is preferable that the binding substance and the aptamer have different sites for binding to the target substance. The binding substance used in the aptamer screening method does not require a separate pairing screening process because the site that binds to the target substance is different from the site that binds to the aptamer target substance, and has excellent sensitivity.

In order to use the binding substance which is not used for the aptamer screening method in the immunoassay method of the present invention, pair screening is required to confirm the binding between the screened aptamer and the binding substance. This is to minimize signal-to-noise generated when the aptamer binds to the binding substance.

Such pair screening may be performed by various published experimental methods, for example, electrophoretic mobility shift assay (EMSAs), titration calorimetry, sedimentation equilibrium assay (e.g., see www.cores.utah.edu/interaction), fluorescence polarization assay, fluorescence anisotropy assay, fluorescence intensity assay, fluorescent resonance energy transition (FRET) assay, nitrocellulose filter binding assay, ELISAs, ELONAs (e.g., see US Patent No. 5,789,163), RIAs, equilibrium dialysis assay, or the like.

However, since such pair screening requires separate effort and time, it is preferable to use the binding substance used in the aptamer screening method of the present invention and the aptamer screened by the screening method in the immunoassay method.

In yet another embodiment of the present invention, the aptamer screening method may further include removing an aptamer that has not formed the complex after the step of forming the complex, or adding an amplification reagent before the step of amplifying the aptamer.

Accordingly, the immunoassay method of the present invention may include mixing the detection sample with the aptamer screened by the aptamer screening method which binds specifically to the target substance to be detected; reacting the mixture with a binding substance immobilized to the support and specifically binding to the target substance to form a complex of aptamer-target substance-binding substance; removing an aptamer that has not formed the complex; and adding an amplification reagent and amplifying the aptamer.

The removing of the aptamer is a washing step and to prevent the aptamer which has not formed the complex of the aptamer-target substance-binding substance from being amplified by the amplification step.

The amplification reagent is a reagent for amplifying the aptamer, and may include a primer that specifically binds to a specific aptamer, dNTP, a reaction buffer, a recombinase, and an intercalating dye inserted into the amplified dsDNA to indicate a fluorescence signal.

The "primer" refers to an oligonucleotide that is a short sequence of nucleotides, and an oligonucleotide which is specifically attached to a complementary position of an opposite strand of a target DNA aptamer to initiate gene amplification, and the primer may be arbitrarily configured in the aptamer screening method of the present invention. Therefore, the aptamer screened by the aptamer screening method of the present invention includes primer nucleotide sequences for aptamer amplification at both terminals and 30 to 50 core sequences in the center, wherein the core sequence includes a sequence specific to the target substance.

The primers preferably include different primer sequences for each target substance, which are for detecting different target substances in one sample.

The recombinase may be derived from prokaryotic, viral or eukaryotic origin. Examples of the recombinase include RecA and UvsX (e.g., RecA protein or UvsX protein obtained from any species), and fragments or mutants thereof, and combinations thereof. The RecA and UvsX proteins may be obtained from any species. In addition, the RecA and UvsX fragments or mutant proteins may also be produced using available RecA and UvsS proteins and nucleic acid sequences, and molecular biology techniques. Exemplary UvsX protein includes proteins derived from myoviridae phages, e.g., T4, T2, T6, Rb69, Aeh1, and KVP40, acinetobacter phage 133, aeromonas phage 65, cyanophage P-SSM2, cyanophage PSSM4, cyanophage S-PM2, Rb14, Rb32, aeromonas phage 25, vibrio phage nt-1, phi1, Rb16, Rb43, Phage 31, phage 44RR2.8t, Rb49, phage Rb3, and phage LZ2. Another exemplary recombinase protein includes archaebacterial RADA and RADB proteins and Rad51 proteins (e.g., RAD51, RAD51B, RAD51C, RAD51D, DMC1, XRCC2, XRCC3, and recA) of eukaryotes (e.g., plants, mammals, and fungi).

The intercalating dye is also referred to as an intercalator as a detectable label for the detection of an amplification product, and does not bind to single-stranded DNA (ssDNA), but binds to double-stranded DNA (dsDNA) formed by extending DNA after primer annealing to emit fluorescence. Therefore, the intercalator fluorescent substance may quantify an amplification product only by measuring the fluorescence in the annealing or DNA synthesis step.

The intercalating dye may be selected from the group consisting of EvaGreen, Alexa Fluor 350, Alexa Fluor 430, Alexa Fluor 488, Alexa Fluor 532, Alexa Fluor 546, Alexa Fluor 568, Alexa Fluor 594, Alexa Fluor 633, Alexa Fluor 647, Alexa Fluor 660, Alexa Fluor 680, Cy2, Cy3.18, Cy3.5, Cy3, Cy5.18, Cy5.5, Cy5, Cy7, Oregon Green, Oregon Green 488-X, Oregon Green 488, Oregon Green 500, Oregon Green 514, SYTO 11, SYTO 12, SYTO 13, SYTO 14, SYTO 15, SYTO 16, SYTO 17, SYTO 18, SYTO 20, SYTO 21, SYTO 22, SYTO 23, SYTO 24, SYTO 25, SYTO 40, SYTO 41, SYTO 42, SYTO 43, SYTO 44, SYTO 45, SYTO 59, SYTO 60, SYTO 61, SYTO 62, SYTO 63, SYTO 64, SYTO 80, SYTO 81, SYTO 82, SYTO 83, SYTO 84, SYTO 85, SYTOX Blue, SYTOX Green, SYTOX Orange, SYBR Green, YO-PRO-1, YO-PRO-3, YOYO-1, YOYO-3 thiazole orange or ethidium bromide, but is not limited thereto.

In the amplifying of the aptamer, known DNA amplification methods including PCR and real-time PCR amplification methods may be used, but in order to detect a selective nucleic acid with high sensitivity in a short time for molecular diagnosis, it is preferable to use isothermal amplification reaction.

The isothermal amplification reaction may be performed by any one method selected from the group consisting of helicase-dependent amplification (HAD), recombinase polymerase amplification (RPA), rolling circle amplification (RCA), loop mediated isothermal amplification (LAMP), nucleic acid sequence-based amplification (NASBA), transcription mediated amplification (TMA), signal mediated amplification of RNA technology (SMART), strand displacement amplification (SDA), isothermal multiple displacement amplification (IMDA), single primer isothermal amplification (SPIA) and circular helicase dependent amplification (cHDA), and preferably recombinase polymerase amplification (RPA).

As a result of performing experiments by a conventional PCR amplification method and an isothermal amplification method under the same conditions, the present inventors confirmed that a fluorescence signal was strongly emitted in the isothermal amplification method compared to the general PCR amplification method (FIG. 8). It is considered that this is because the high reaction temperature of the general PCR amplification method affects the detection efficiency of the intercalating dye.

In yet another embodiment of the present invention, a step of measuring fluorescence after the step of amplifying the aptamer may be further included. The fluorescence measuring step is to confirm whether the aptamer is amplified, and the intercalating dye may bind to the amplified dsDNA of the aptamer, and the fluorescence of a specific wavelength emitted by the intercalating dye is measured to determine whether the aptamer is amplified. Since the wavelength of fluorescence emitted by each intercalating dye is different, it is preferable to measure the fluorescence of a specific wavelength emitted by the above-listed intercalating dyes.

In the embodiment, EvaGreen was used as the intercalating dye, and the fluorescence signal was measured at a wavelength of λabs/λem = 500/530 nm. However, the measured wavelength may vary depending on an intercalating dye to be added to the amplification reagent.

In a specific embodiment of the present invention, the concentration of the binding substance is 0.1 to 100 ng/mL, the concentration of the aptamer is 0.01 to 10 pM, and the step of amplifying the aptamer may be performed for 8 to 30 minutes, but is not limited thereto.

The concentration of the binding substance may be 0.1 to 100 ng/mL, preferably 0.1 to 10 ng/mL, and according to Experimental Example of the present invention, the highest relative fluorescence intensity was exhibited in the case of using 1 ng/mL.

The concentration of the aptamer may be 0.01 to 10 pM, preferably 0.1 to 10 pM, and according to Experimental Example of the present invention, the highest relative fluorescence intensity was exhibited in the case of using 1 pM.

The intercalating dye is preferably used at a concentration obtained by diluting the concentration of a stock solution 20 to 40 times.

The amplification of the aptamer is preferably performed for 8 to 30 minutes, 8 to 20 minutes, 8 to 15 minutes, or 8 to 10 minutes. According to Experimental Example of the present invention, the fluorescence signal increased rapidly for 8 to 10 minutes, and the reaction time was saturated at 15 minutes.

The lower limits of detection (LOD) of the immunoassay method using the aptamer is 1 fg/mL or 1 fg/mL or more of the concentration of the target substance contained in the sample, which represents the lowest limits of detection (LOD) compared to various known immunoassay methods.

[Advantageous Effects]

According to the present invention, the aptamer screened by the screening method binds to a site other than a site where an antibody binds to a target substance to be applicable in various fields such as sandwich-type biosensors and reduce a significant amount of time without requiring a separate pairing selection process. In addition, such an aptamer has excellent stability and sensitivity compared to conventional preparations comprising an antibody, can be mass-produced at low cost in a short time by a chemical synthesis method, and is easily transformed in various ways to increase a binding force.

In addition, the immunoassay method using the aptamer screened by the aptamer screening method of the present invention selectively amplifies only the aptamer binding to the target substance in a heterogeneous sandwich structure to detect a relative fluorescence signal, thereby detecting the target substance sensitively and quickly.

[Description of Drawings]

FIG. 1 illustrates a result of confirming the size of a purified nuclear protein of severe fever with thrombocytopenia syndrome (SFTS) virus through SDS-PAGE according to an embodiment of the present invention.

FIG. 2 illustrates a schematic diagram of each step of a modified SELEX method according to an embodiment of the present invention.

FIGS. 3A to 3C are graphs showing a result of confirming a target binding force of an aptamer screened according to a modified SELEX method according to an embodiment of the present invention.

FIG. 4 illustrates a result of confirming binding specificity to nuclear proteins of SFTS virus according to an embodiment of the present invention.

FIGS. A and B illustrate a result of confirming a binding force of an aptamer according to a nuclear protein concentration of SFTS virus according to an embodiment of the present invention.

FIG. 6 illustrates schematically a principle of an immunoassay method (H-sandwich RPA) using an aptamer screened by an aptamer screening method of the present invention, wherein a sample containing an antigen and an aptamer is pre-incubated in a well immobilized with a capture antibody. The aptamer binding to the antigen is amplified during an RPA reaction and detected by an intercalating dye.

FIG. 7 is a graph showing optimization results of factors affecting an immunoassay method (H-sandwich RPA) using an screened aptamer, wherein FIG. 7A illustrates a concentration of a coating antibody used for preparing an immobilized well, FIG. 7B illustrates a concentration of an aptamer reacting with an antigen, FIG. 7C illustrates a concentration of an intercalating dye, and FIG. 7D illustrates relative fluorescent strength saturation for an RPA reaction, and concentrations of an influenza A NP and an aptamer were set at 0.5 pg/mL and 1 pM, respectively.

FIG. 8 illustrates intercalating dye efficiency under various amplification conditions, wherein FIG. 8A illustrates relative fluorescence intensity through a amplification reaction (a: conventional PCR (30 cycles), b: RPA, c: H-sandwich RPA(NP and aptamer are 0.5 pg/mL and 1 pM, respectively), and FIG. 8B illustrates a gel shift image under each amplification reaction (OFF: no aptamer or NP, ON: aptamer 1 pM, NP).

FIG. 9 illustrates results of measuring target concentrations using an immunoassay method (H-sandwich RPA) using a screened aptamer, wherein FIG. 9A illustrates influenza A NP, FIG. 9B illustrates influenza B NP, FIG. 9C illustrates HIV-1 p24, FIG. 9D illustrates Ebola NP, and FIG. 9E illustrates SARS-Cov-2 NP. Each antigen is in a concentration range of 0.001 to 10 pg/mL.

FIG. 10 is a graph showing specificity of H-sandwich RPA based on target-aptamer specificity, wherein FIG. 10A illustrates cross-reactivity between an influenza A NP and an influenza B NP (Blue bar: influenza A NP-specific aptamer 1 pM, influenza A and B NPs were applied at 1 and 10 pg/mL, respectively, Green bar: influenza B NP-specific aptamer 1 pM, influenza A and B NPs were applied at 10 and 1 pg/mL, respectively), and FIG. 10B illustrates cross-reactivity of p24 and Ebola NP (Orange bars: p24-specific aptamer 1 pM, p24 and Ebola NPs were applied at 1 and 10 pg/mL, respectively, Pink bar: Ebola NP-specific aptamer 1 pM, p24 and Ebola NP are applied at 10 and 1 pg/mL, respectively).

[Modes for the Invention]

Hereinafter, the present invention will be described in more detail with reference to Examples. These Examples are to explain the present invention in more detail, and it will be apparent to those skilled in the art that the scope of the present invention is not limited by these Examples in accordance with the gist of the present invention.

**<Example 1> Screening method of aptamer**

**1. Design of single strand DNA aptamer (ssDNA) library**

In an aptamer screening method, in an initial round, an ssDNA oligonucleotide library randomly containing 40 nucleotide sequences (SEQ ID NO: 2: 5'-ATC CAGAGT GAC GCA GCA -[core sequence; (N X 40)]- TG GAC ACG GTG GCT TAG T -3') was used. All oligonucleotides used in this study were obtained from Integrated DNA Technologies Inc. (Coralville, IA, USA). At this time, the ssDNA library was put in a binding buffer (50 mM Tris-hydrochloric acid, pH 7.4, 100 mM NaCl, 5 mM KCl, 1 mM $MgCl_2$), denatured by heating at 90°C for 5 minutes, and then washed with 0.01% Tween 20, and immediately cooled on ice for 10 minutes, and then used.

**2. Expression and purification of nuclear protein of server fever with thrombocytopenia syndrome (SFTS) virus**

A PCR product was obtained by amplifying a nucleotide sequence encoding a protein consisting of an amino acid sequence represented by SEQ ID NO: 1 through a polymerase chain reaction (PCR). Thereafter, the PCR product was inserted into a pET28a expression vector and then transformed to E.Coli. BL21 and shake-incubated at 37°C. When an $OD_{600}$ value of a shake-culture medium reached 0.6, 1 mM isoprophyl-b-D-thiogalactopyranoside (IPTG) was treated and incubated overnight at 25°C to induce protein expression. Thereafter, the transformant was applied with ultrasonic waves to destroy cells, and then a protein represented by SEQ ID NO: 1 was obtained from the cell lysate.

As illustrated in FIG. 1, as a result of performing SDS-PAGE on the isolated protein according to a general method, it was confirmed that a nuclear protein of server fever with thrombocytopenia syndrome (hereinafter referred to as 'SFTS') virus, as a protein consisting of the amino acid sequence represented by SEQ ID NO: 1 was obtained.

**3. Screening method of aptamer**

**(1) Each step of screening method of aptamer**

As illustrated in FIG. 2, the screening method of the aptamer of the present invention was performed to screen an aptamer capable of specifically binding to the SFTS virus. Specifically, the screening method of the modified aptamer was as follows.

**1) Antibody immobilizing and blocking steps**

Antibody immobilizing step: The antibody specifically binding to the amino acid sequence represented by SEQ ID NO: 1 was diluted in a 0.1 M carbonate buffer in pH 9.5, put in a 96-well plate and then reacted overnight at 4°C to obtain the antibody on the 96-well plate.

Blocking step: Thereafter, 200 μl of a washing buffer (potassium phosphate buffer (PBS) containing 0.01% Tween) was added and washed in the coated plate. Non-specific binding reaction that may occur when ssDNA bound to the antibody was blocked by adding 1% bovine serum albumin (BSA) in the washed plate and reacting for 1 hour at room temperature.

1-1) Recovery step;

In step 1), in order to reduce non-specific binding possibility between a protein except for the amino acid sequence represented by SEQ ID NO: 1 and ssDNA, a recovery step was additionally performed, in which a buffer including the ssDNA library was added to the blocked plate using the BSA and incubated for 30 minutes, and then a supernatant (hereinafter, referred to as a 'supernatant') containing only the ssDNA library without binding to the antibody was obtained.

**2) First reaction step; antigen-antibody reaction**

The protein of Example 1 diluted in PBS at a concentration of 1 μg/mL was added to the plate on which the antibody immobilization and blocking were completed in step 1), and incubated at room temperature for 2 hours.

**3) Second reaction step; step of binding to ssDNA library**

The supernatant of 1-1) was added to the plate on which the first reaction step was completed, reacted at room temperature for 1 hour, and then washed 5 times using 200 μl of a wash buffer.

**4) First elution step; ssDNA elution and amplification step**

A binding buffer was added to the plate washed in step 2), and reacted at 80°C for 10 minutes to elute ssDNA bound to the protein of Example 1.

**5) Amplification step; Third reaction step; and Second elution step**

The ssDNA eluate of the first elution step 4) was purified according to a method provided by a manufacturer using a PCR purification kit, and then the purified ssDNA was dissolved in 30 μl of sterile water. The purified ssDNA was mixed with 1 μM of a primer pair, a 2.5 mM dNTP mixture, and 1.2 U of Pfu polymerase to make a final volume of 50 μl, and then polymerase chain reaction was performed to obtain amplified ssDNA. At this time, in the case of the polymerase chain reaction, 25 cycles were performed under conditions of 5 min at 95°C; 30 sec at 95°C; 20 sec at 57°C; and 20 sec at 72°C and the final extension was performed under a condition of 5 min at 72°C.

Then, the amplified ssDNA was subjected to a third reaction step in the same manner as in the '3) second reaction step', and subjected to a second elution step in the same manner as in the '4) first elution step', and the amplification step; the third reaction step and the second elution step were performed at least 10 times. Through this process, three ssDNA sequences were finally screened.

**(2) ssDNA sequencing**

The three types of ssDNA sequences selected by the aptamer screening method were amplified through the same polymerase chain reaction as in the aptamer screening method, and the amplified products were inserted into the pETHis6 TEVLIC cloning vector (Addgene, USA), respectively. Then, after transforming the vector into E.Coli DH5α, the amplified plasmid was isolated from the transformant to perform DNA sequencing. Here, the DNA sequencing was performed in Macrogen (Korea), and the secondary structure of the aptamer as ssDNA was predicted using an Mfold program based on a Zuker algorithm.

As a result, the screened aptamer had a nucleotide sequence of 5'- ATC CAG AGT GAC GCA GCA -[core sequence; (N X 40)]- TG GAC ACG GTG GCT TAG T -3' of SEQ ID NO: 2 and the nucleotide sequence was the same as described in Table 1 below.

[Table 1]

| Name | SEQ ID NO: | Core sequence |
|---|---|---|
| Aptamer 1 | SEQ ID NO: 3 | CGACCACAGATTGGAGACTGATAGTGCA CGAGCAAGGACA |

(continued)

| Name | SEQ ID NO: | Core sequence |
|---|---|---|
| Aptamer 2 | SEQ ID NO: 4 | TCGGATGGATTGTGGTCGAAGTTGTTTC CGACACTAGTCA |
| Aptamer 3 | SEQ ID NO: 5 | CACATCGGAGAACAGGCGCACTGTCGG AGGAACCGCAACG |

A phosphor (FAM) (in the case of FAM-labeled aptamers, hereinafter referred to as 'FAM-aptamer 1', 'FAM-aptamer 2' and 'FAM-aptamer 3') or biotin was labeled on terminals of the sequences of the screened three types of aptamers according to a general method so that a binding force may be measured and observed due to a change in polarization of the phosphor according to a binding force to the target.

**4. Confirmation of target binding force of screened aptamer**

After the FAM-aptamer (20 nM) was coated on a 384-well flat-bottom plate, the concentration of a target protein, SFTS virus nuclear protein (NP), was added to the plate at a concentration of 0 to 25 μM and shaken for 5 minutes, and then incubated at room temperature for 30 minutes. Then, the expression intensities at an excitation wavelength of 480 nm and an emission wavelength of 535 nm were measured using a Cytation 5.0 Plate Reader (BioTek) and Gen5 version 2.09.1, and an anisotropy value was plotted against an X-axis with an increasing concentration and then a dissociation constant Kd was measured using a saturation one-site fitting model, and the results were shown in FIGS. 3Ato 3C.

As illustrated in FIGS. 3A to 3C, it was confirmed that the dissociation constant of FAM-aptamer 1 was 3.9 μM, the dissociation constant of FAM-aptamer 2 was 1.8 μM, and the dissociation constant of FAM-aptamer 3 was 0.8 μM.

From the results, it can be seen that the three types of aptamers screened according to the present invention have the difference above, but all have excellent binding affinity, and in particular, in the case of FAM-aptamer 3, the binding affinity is very excellent. Furthermore, it can be seen from these results that the modified SELEX method according to the present invention is very effective in screening excellent aptamers.

**5. Confirmation of antigen specificity of screened aptamers**

A serum containing another type of hantavirus (Hanta) or influenza A virus (InfA) similar in structure to the nuclear protein of SFTS, a serum containing the nuclear protein of SFTS, and a serum (Mix) combined with these serums were added to a 96-well plate coated with a biotin-labeled aptamer-3 (hereinafter, 'biotin-aptamer 3'), respectively, and added with Streptavidin bound with HRP, and then reacted, and thereafter, color changes were confirmed and the result was illustrated in FIG. 4.

In addition, the biotin-aptamer 3 was applied to a conventional liposome-based colorimetric sensor platform to determine whether a target was detected, and the result thereof was shown in FIGS. 5A and 5B.

As illustrated in FIG. 4, it was confirmed that the absorbance was 0.1 a.u. in the serum containing only the Hanta virus or influenza A virus, which had a similar structure to the nuclear protein of SFTS, whereas in the serum containing the nuclear protein of SFTS and the mixed serum, the absorbance was 0.7 a.u.

In addition, as illustrated in FIGS. 5A and 5B, it was confirmed that as the concentration of the target protein was increased, not only the intensity of the color to be developed was increased, but also the absorbance was also increased in a concentration-dependent manner.

Through the result, it can be seen that the aptamer according to the present invention has binding specificity capable of specifically binding only to the nuclear protein of SFTS, and as the binding degree varies depending on the concentration of the target protein, the concentration of the target protein present in a desired serum may be measured with high sensitivity.

**<Example 2> Immunoassay using screened aptamer**

**1. Screening of target-specific aptamer**

The present inventors commercially purchased influenza A NP, influenza B NP, HIV-1 p24 protein, Ebola NP, and SARS-Cov-2 NP, and screened aptamers having binding specificity to each protein by the method of Example 1, and the sequences of the screened aptamers were shown in Table 2. In Table 2 below, the sequences indicated in bold were sequences bound with primers in the aptamer amplification step, and were prepared by different sequences for each aptamer.

[Table 2]

| Protein | SEQ ID NO: | Aptamer sequence |
|---|---|---|
| Influenza A NP | SEQ ID NO: 6 | 5' **TAG GGA AGA GAA GGA CAT ATG** ATG GCG TAC GGG GAT GAG GTG ATC GTA GTG GG**T TGA CTA GTA CAT GAC CAC TTG A** 3' |
| Influenza B NP | SEQ ID NO: 7 | 5' **ATT ATG GCG TTT GCA GCG TTC TGG TT**G GTG GTG GTG ATA GGT GGG GGG AAG GAG GGT **ATC TTG TTG GTG AGG TAA CGG CT** 3' |
| HIV-1 p24 protein | SEQ ID NO: 8 | 5' **AGA TAC TGC CAT TCA TTG CAT C**GA GCA CGC GAC TGA TGA GGA TGG TCT AGT AGC TGG GGT **CGA GTA CTA AGC TAT GTG TCG A** 3' |
| Ebola NP | SEQ ID NO: 9 | 5' **GAT GTG AGT GAC GTG GAT CGA G**CG GAT GTG AAG GCT GAA AGT GGC TTT GGG CGG TCG TAA GT**G TCA CAG AGC ATG CAA CAA GAC C** 3' |
| SARS-Cov-2 NP | SEQ ID NO: 10 | 5' **ATC CAG AGT GAC GCA** GCA AAC CCA AGC AAA CTA CCT CTA TAC CCT TCG ACC TTC ATC A**TG GAC ACG GTG GCT TAG T** 3' |

**2. Heterogeneous sandwich immunoassay using screened aptamer**

The present inventors examined the detection efficiency of H-sandwich RPA by detecting various concentration ranges of five model target proteins using the selected aptamers (FIG. 6).
Anti-target antibody (1 ng/mL) in a 0.1 M carbonate buffer (pH 9.6) was immobilized in a 96-well microplate at 4°C overnight. The coated wells were washed 3 times with PBS-T and blocked with 1% BSA (w/w). Recombinant target proteins and aptamers (1 pM) at various concentrations were pre-incubated at room temperature for 30 minutes. For clinical sample analysis, a binding buffer containing 2% Triton™ X-100 as an assay diluent to lyze the virus was used. The target protein-aptamer mixture was loaded into the coated wells and incubated for 45 minutes at room temperature while gently shaking. An RPA solution containing a primer, dNTP, a reaction buffer and magnesium acetate (MgOAc) was added to the H-sandwich complex containing the antibody-antigen-aptamer to induce isothermal amplification, and an intercalating dye (0.5 × EvaGreen®) to facilitate detection of a fluorescence signal was added to the wells and incubated at 37°C for 20 minutes. Thereafter, the fluorescence signal was measured at an excitation wavelength of 500 nm in an emission wavelength of 530 nm. The fluorescence intensity of the reaction without the target protein was used as a negative control group (Ic), and the fluorescence signal after the RPA reaction with the target protein was recorded as a fluorescence intensity (I). Normalized ratios of fluorescence intensities were calculated using a difference in signal values as follows:

$$\text{Relative fluorescence intensity or } \Delta I = (I - Ic)/Ic.$$

**3. Optimization of heterogeneous sandwich immunoassay**

H-sandwich RPA was applied to optimize conditions of factors affecting the detection efficiency to detect a model target (FIGS. 7A to 7D).

**(1) Concentration of antibody and aptamer**

First, the detection efficiency according to the concentration of capture antibodies immobilized in the wells and the concentration of the aptamers was examined. The concentrations of the capture antibodies and the aptamers were one of main factors of H-sandwich RPA, and this was because an appropriate concentration of antibodies needs to be immobilized in the wells to capture the antigens and amplify only the antigen-bound aptamers through the RPA reaction.
As illustrated in FIG. 7A, when the antibody was immobilized at a concentration of 0.1 to 100 ng/mL, and 1 ng/mL of antibody was added while 1 pM of the aptamer and 0.5 pg/mL of influenza A NP reacted with each other, the highest relative fluorescence intensity was shown.
Next, the concentration conditions of the aptamer reacting with the antigen were examined (FIG. 7B).
Influenza A NP 0.5 pg/mL was used, and the experiment was performed under aptamer conditions of 0.01 to 10 pM, which was 1 to 1000 times larger than the antigen concentration. The highest relative fluorescence intensity was observed at 1 pM of the aptamer, which was 100 times larger than the antigen concentration.
Therefore, a subsequent experiment was performed under conditions of 1 ng/mL of the coated antibody and 1 pM of the aptamer.

**(2) Concentration of intercalating dye**

In addition, by examining a dilution ratio of an intercalating dye, the concentration of the dye representing the highest relative fluorescence intensity value in the on-off of the antigen was determined. The reacted antigen concentration was 0.5 pg/mL and the aptamer used was 1 pM.
The intercalating dye was diluted from a 20X stock to 0.05, 0.1, 0.5 and IX, and the measured fluorescence signals were illustrated In FIG. 7C.
The fluorescence intensity was increased according to a higher dilution ratio, and when 0.5X intercalating dye was added, the highest relative fluorescence intensity was exhibited.
Therefore, in an additional experiment, the dilution ratio of the intercalating dye was set to 0.5X.

**(3) Saturation time of fluorescence intensity**

Finally, the present inventors examined the saturation time of the fluorescence intensity generated by the intercalating dye during the RPA reaction.
As illustrated in FIG. 7D, the fluorescence signal increased rapidly for 8 to 10 minutes, and the reaction time was saturated at 15 minutes. In general, the RPA reaction showed a slight time and a difference in fluorescence signal intensity depending on the enzyme working efficiency, and 20 minutes, which were a recommended condition of an RPA kit, was selected to compensate for these the time and the difference.

**(4) Aptamer amplification method**

In addition, the dsDNA detection efficiency of the intercalating dye used in the present invention was confirmed.
To determine whether the intercalating dye added to the amplification reaction showed increased fluorescence intensity with the dsDNA generated by the amplification process, normal PCR, solution RPA and H-sandwich RPA were performed. The amounts of primers, aptamers and other reagents used in each amplification reaction were the same as each other, normal PCR was performed in 30 cycles, and the RPA reaction time at 37°C was 20 minutes.
The primers used for the amplification reaction were shown in bold in Table 2, and specifically, a forward primer was the same as a sequence shown in bold in Table 2 and a reverse primer had a sequence complementary to the sequence shown in bold in Table 2.
As illustrated in FIG. 8, a difference in fluorescence signal before and after amplification occurred in each method, and the relative fluorescence intensity was highest in the solution RPA, but was not significantly different from that of the H-sandwich RPA. In each case, the amplified DNA was confirmed through gel electrophoresis, and a band difference according to the presence or absence of the antigen was clearly observed in the H-sandwich RPA (FIG. 8B).
In the normal PCR, the high amplification reaction temperature may affect the detection efficiency of the intercalating dye, and since the H-sandwich RPA amplifies the aptamers on the immune complex bound to the wells, it is supposed that the on-off difference on the gel is obvious.

**4. Effects of immunoassay under optimal conditions**

In order to examine the effects of the immunoassay of the present invention to which the optimized conditions were applied, fluorescence signals after RPA reaction were measured using model targets and target-specific aptamers in various concentration ranges.
After the capture antibody of each target was immobilized in the well, premixed antigens and aptamers were loaded into the wells and incubated to form immune complexes. After the heterogeneous sandwich immune complex (antibody-antigen-aptamer) was constructed, unbound aptamers were removed through a sufficient washing step. Thereafter, the primers, the RPA reagents and the intercalating dye were added to the wells and incubated at 37°C for 20 minutes to induce the RPA reaction and fluorescence signals generated from the amplified aptamers were measured.
As illustrated in FIG. 9, the fluorescence signal of the influenza A virus NP was detected at a concentration of 1 fg/mL, which was confirmed by observing a band corresponding to the aptamer length on an agarose gel.
In the same procedure as described above, the present inventors also applied the detection method of the present invention to the detection of other virus-related antigens including influenza B virus NP, HIV-1 virus p24, Ebola virus NP, SARS-Cov-2 NP, respectively.
In addition to the fluorescence intensity measurement, gel bands were observed in the concentration range of 1 and 10 fg/mL (not illustrated), and the relative fluorescence intensity value of the detection limit concentration was 0.5 or less (FIGS. 9B to 9E). As a result of comparing the relative fluorescence intensity of each target with a gel electrophoresis image, no bands were observed in a control group (no target antigen) with a fluorescence signal of less than 0.5 and concentration groups.

**5. Crossover possibility depending on interfering substrate**

Next, the crossover possibility of the H-sandwich RPA platform according to the presence of an interfering substance was examined.
The relative fluorescence intensity was measured in a mixture of influenza A, B virus NPs or p24, Ebola virus NP and human fluid according to the use of each target-specific aptamer (FIG. 10). The relative fluorescence intensity was calculated based on the fluorescence signal detected after performing an experiment by the above-mentioned method in each well divided into groups containing 10-fold diluted human nasal fluid, target or non-target, and target and non-target.
The relative fluorescence intensity of each group was corrected using the fluorescence intensity of the control well (Ic). In the case of using an aptamer specific to influenza A NP, the target was influenza A NP and the non-target was influenza B NP (blue bar). The target and the non-target were vice versa when an influenza B NP-specific aptamer (pink bar) was used.
As illustrated in FIG. 10A, THE high relative fluorescence intensity was observed in the presence of the target despite the 10-fold higher non-target concentration, and there was little matrix effect in the diluted nasal fluid containing non-specific DNA and protein. When p24 and Ebola NP-specific aptamers were used as detection probes in 10-fold diluted serum, target, non-target and mixture, respectively, high relative fluorescence intensities were also shown in the presence of the target (FIG. 10B).

These results prove that the H-sandwich RPA has high selectivity and sensitivity due to its high signal-to-noise ratio. The immunoassay method of the present invention may maximize selectivity by using a target-specific capture antibody and an aptamer, and amplifying only the DNA aptamer in the formed heterogeneous sandwich immune complex. These properties of the proposed method were distinguished from those of conventional immunoassays with low signal-to-noise ratios using the same two bioreceptors.

**6. Comparison with various immunoassay methods**

The present inventors compared the H-sandwich RPA of the present invention with various immunoassay methods.

[Table 1]

| Detection method | Target | LOD | Multiple detection |
|---|---|---|---|
| PCR using short DNA aptamers | Thrombin | 2 pM | Impossible |
| Sandwich ELISA using DNA encapsulated liposomes | Protective antigen | 4.1 ng/mL | Possible |
| Photoelectrochemical immunoassay using DNA labeling | HIV-1 p24 | 10 ng/mL | Impossible |
| H-sandwich RPA using DNA aptamers | Virus-related protein | to 1 fg/mL | Possible |

Referring to Table 1, the immunoassay method of the H-sandwich RPA enables multiple detection and showed the lowest limit of detection (LOD) compared to other immunoassays. Therefore, the immunoassay method of the present invention has an advantage of detecting the target substance present at a low concentration of 1 fg/mL by selectively amplifying only the aptamer bound to the target substance in the heterogeneous sandwich structure to detect the relative fluorescence signal.

**<Conclusion>**

The present inventors have developed a highly sensitive immunoassay method for the detection of protein biomarkers based on the integration of RPA and immunoassay. The present inventors also newly selected DNA aptamers specific to HIV-1 p24, Ebola virus NP and SARS-Cov-2 NP by the aptamer screening method of the present invention. Unlike immuno-PCR using capture and detection antibody pairs, since the H-sandwich RPA uses a DNA aptamer that binds to the antibody-antigen complex in an H-sandwich form, antibody pair screening is not required. The H-sandwich RPA has an advantage of high sensitivity and specificity due to the rapid amplification of target-bound aptamers through the RPA reaction. Through the application of the proposed immunoassay method and optimization of various reagents, a low limit of detection for H-sandwich RPA was observed with the detection of an attomolar concentration level of a model target protein. This method showed higher detection efficiency for influenza-infected patient samples than commercial ELISA and LFA kits. The approach of the present inventors enables high-sensitivity detection of protein biomarkers in a well-plate, unlike conventional PCR-based amplification methods that may be applied only to nucleic acids. Therefore, the results of the present inventors suggest that the H-sandwich RPA may be universally applied to various targets through appropriate selection of biomarker-specific aptamers.

In the prior art, Fischer, Nicholas O et al., there is disclosed a technique of immobilizing an anti-protein antibody or a biotinylated protein target to magnetic beads, binding to the aptamer and the amplifying the bound aptamer through PCR. However, in the prior art, the aptamer is separated using magnetic beads and transferred to a tube to perform the amplification reaction. Unlike this, the present invention may be differentiated from the prior art in that aptamer binding, amplification, and detection are enabled on one plate, and multiplex PCR-based detection is enabled by using a target-specific aptamer.

## Claims

1. A screening method of an aptamer comprising:

   an antibody immobilization step of immobilizing a specific antibody to a target substance to a support;
   a first reaction step of adding and reacting the target substance to the antibody-immobilized support;
   a second reaction step of adding and reacting an aptamer library to the support where the first reaction step is completed; and
   a first elution step of eluting the aptamer binding to the target substance in the second reaction step.

2. The screening method of the aptamer of claim 1, further comprising:

   an amplification step of amplifying a nucleic acid of the aptamer eluted in the first elution step;
   a second-1 reaction step of adding and reacting the amplified nucleic acid of the aptamer into the support on which the first reaction step has been completed; and
   a second elution step of eluting the aptamer binding to the target substance in the second-1 reaction step.

3. The screening method of the aptamer of claim 2, wherein the amplification step is performed through a polymerase chain reaction.

4. The screening method of the aptamer of claim 2, wherein the amplification step; the second-1 reaction step; and the second elution step may be repeated 2 to 30 times in sequence.

5. The screening method of the aptamer of claim 1, further comprising:

   after the first reaction step, a step of adding and reacting the aptamer library to the support immobilized with the antibody; and
   a recovery step of recovering a supernatant containing the aptamer library that has not reacted with the antibody.

6. The screening method of the aptamer of claim 1, wherein the aptamer library includes at least one single-stranded nucleotide sequence selected from the group consisting of different nucleotide sequences.

7. The screening method of the aptamer of claim 6, wherein the single-stranded nucleotide sequence consists of a forward or reverse primer nucleotide sequence for amplification at both terminals, and the center of the primer nucleotide sequence consists of 30 to 50 nucleotide sequences.

8. The screening method of the aptamer of claim 1, wherein the target substance is at least one selected from the group consisting of metal ions, compounds, nucleic acids, proteins, peptides and cells.

9. An aptamer screened by the screening method of any one of claims 1 to 8.

10. An immunoassay kit comprising the aptamer of claim 9.

**11.** An immunoassay method using the aptamer of claim 9.

**12.** An immunoassay method comprising:

mixing a detection sample with an aptamer specifically binding to a target substance to be detected;
reacting the mixture with a binding substance immobilized to the support and specifically binding to the target substance to form a complex of aptamer-target substance-binding substance; and
amplifying the aptamer.

**13.** The immunoassay method of claim 12, wherein the aptamer is the aptamer of claim 9.

**14.** The immunoassay method of claim 12, further comprising:
removing an aptamer that has not formed the complex after the step of forming the complex.

**15.** The immunoassay method of claim 12, further comprising:
adding an amplification reagent before the step of amplifying the aptamer.

**16.** The immunoassay method of claim 12, further comprising:
measuring fluorescence after amplifying the aptamer.

**17.** The immunoassay method of claim 12, wherein the binding substance is at least one selected from the group consisting of antibodies, antigens, nucleic acids, aptamers, hapten, antigen proteins, DNA, RNA binding proteins, and cationic polymers.

**18.** The immunoassay method of claim 15, wherein the amplification reagent includes a primer, dNTP, a reaction buffer, a recombinase, and an intercalating dye inserted into the amplified dsDNA to indicate a fluorescence signal.

**19.** The immunoassay method of claim 12, wherein the amplifying of the aptamer is performed by any one isothermal amplification method selected from the group consisting of helicase-dependent amplification (HAD), recombinase polymerase amplification (RPA), rolling circle amplification (RCA), loop mediated isothermal amplification (LAMP), nucleic acid sequence-based amplification (NASBA), transcription mediated amplification (TMA), signal mediated amplification of RNA technology (SMART), strand displacement amplification (SDA), isothermal multiple displacement amplification (IMDA), single primer isothermal amplification (SPIA) and circular helicase dependent amplification (cHDA).

**20.** The immunoassay method of claim 12, wherein the concentration of the binding substance is 0.1 to 100 ng/ml.

**21.** The immunoassay method of claim 12, wherein the concentration of the aptamer is 0.01 to 10 pM.

**22.** The immunoassay method of claim 12, wherein the amplifying of the aptamer is performed for 8 minutes to 30 minutes.

**23.** The immunoassay method of any one of claims 12 to 22, wherein a low limit of detection (LOD) of the method is that the concentration of the target substance contained in the sample is 1 fg/mL or more.

[FIG. 1]

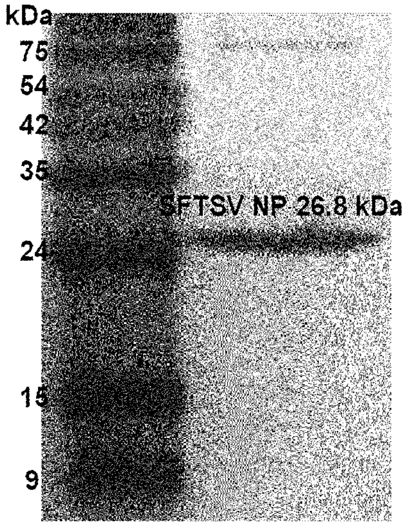
kDa
75
54
42
35
SFTSV NP 26.8 kDa
24
15
9

[FIG. 2]

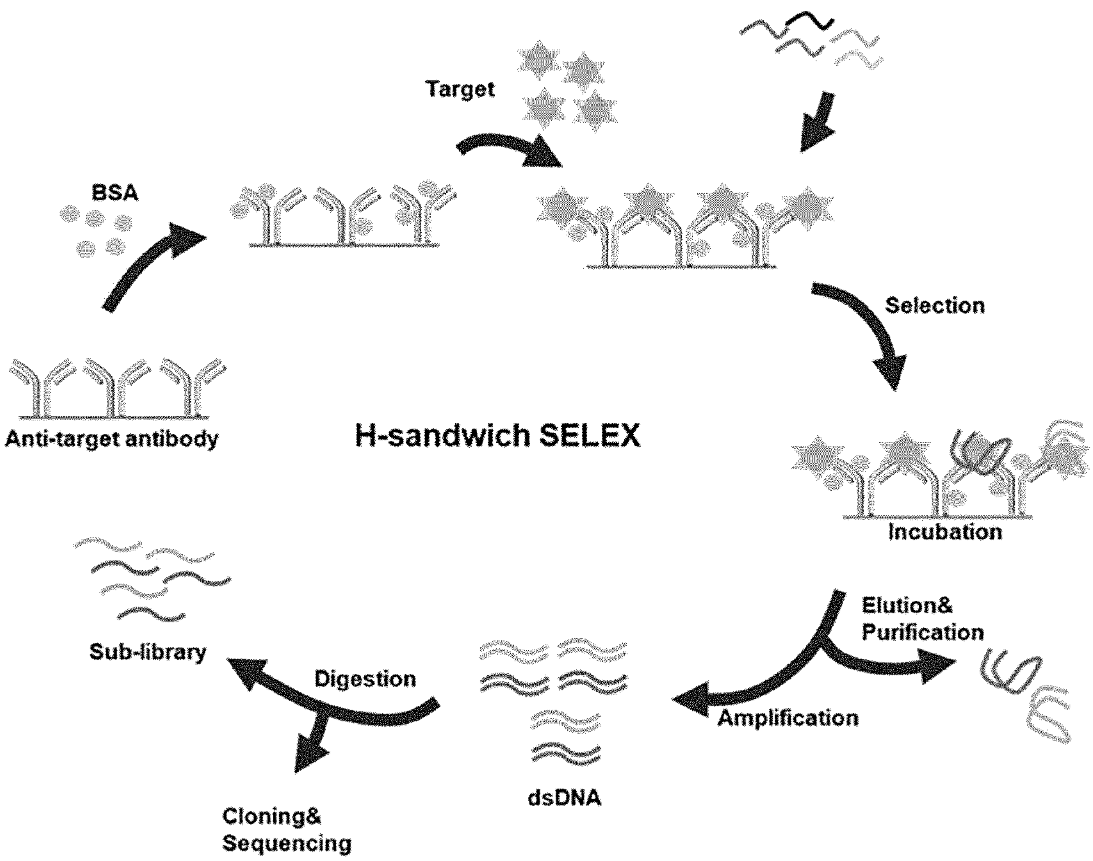
Target
BSA
Selection
Anti-target antibody
H-sandwich SELEX
Incubation
Elution&
Purification
Sub-library
Digestion
Amplification
dsDNA
Cloning&
Sequencing

[FIG. 3]

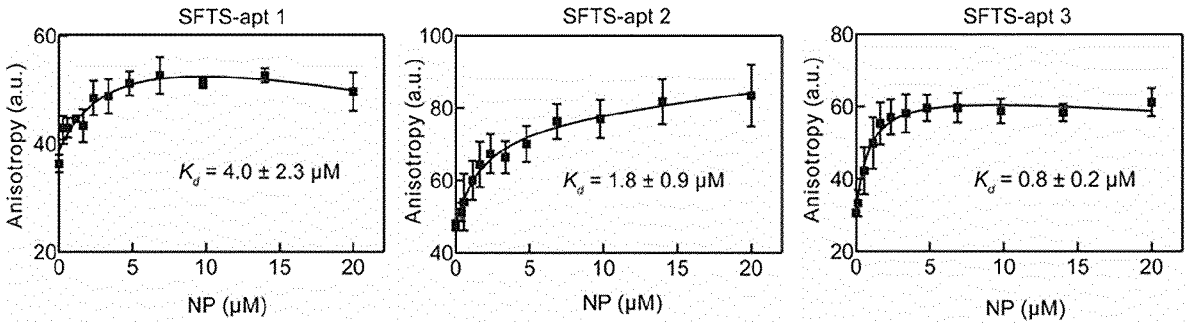
SFTS-apt 1
Anisotropy (a.u.)
NP (μM)
$K_d$ = 4.0 ± 2.3 μM
SFTS-apt 2
Anisotropy (a.u.)
NP (μM)
$K_d$ = 1.8 ± 0.9 μM
SFTS-apt 3
Anisotropy (a.u.)
NP (μM)
$K_d$ = 0.8 ± 0.2 μM

[FIG. 4]

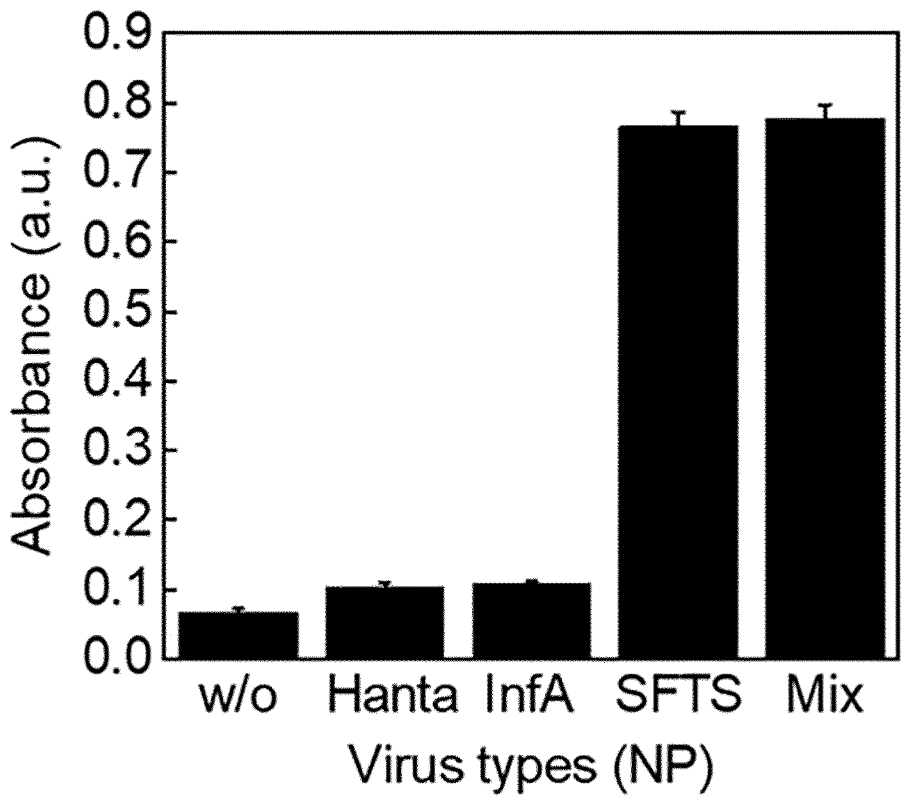
Absorbance (a.u.)
w/o
Hanta
InfA
SFTS
Mix
Virus types (NP)

[FIG. 5]

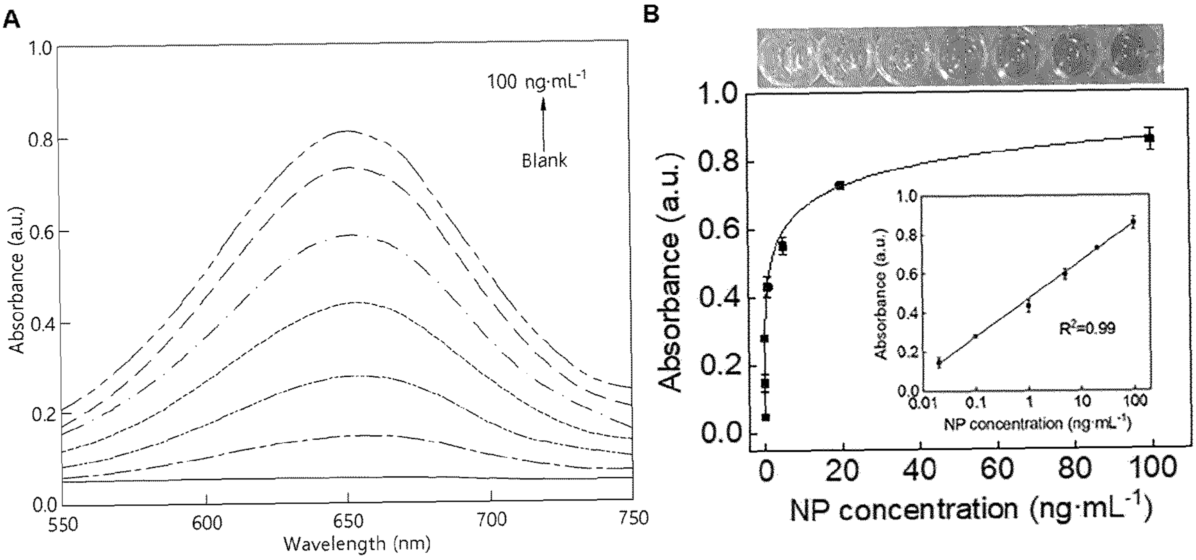
A
100 ng·mL-1
Blank
Absorbance (a.u.)
Wavelength (nm)
B
Absorbance (a.u.)
NP concentration (ng·mL-1)
R2=0.99

[FIG. 6]

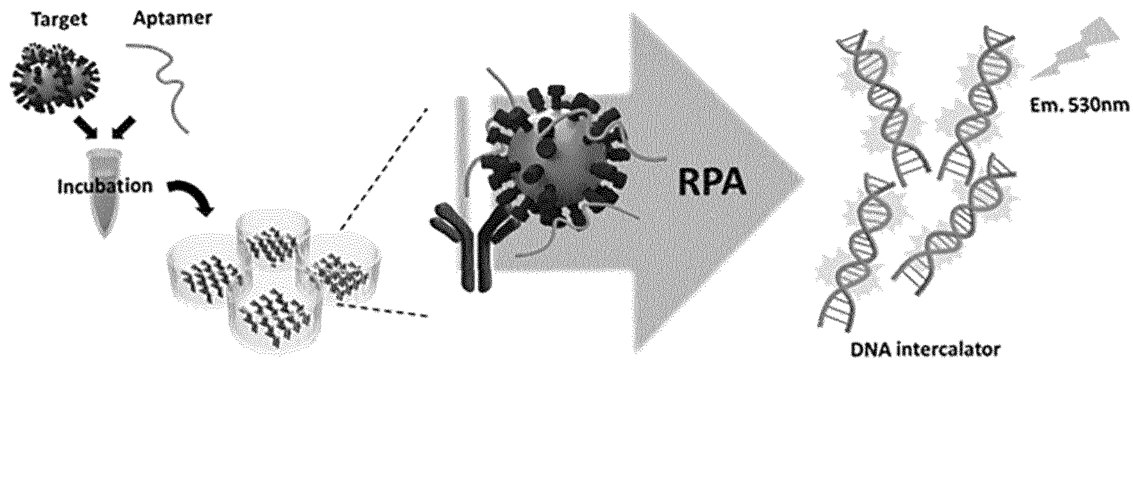
Target
Aptamer
Incubation
RPA
Em. 530nm
DNA intercalator

[FIG. 7]

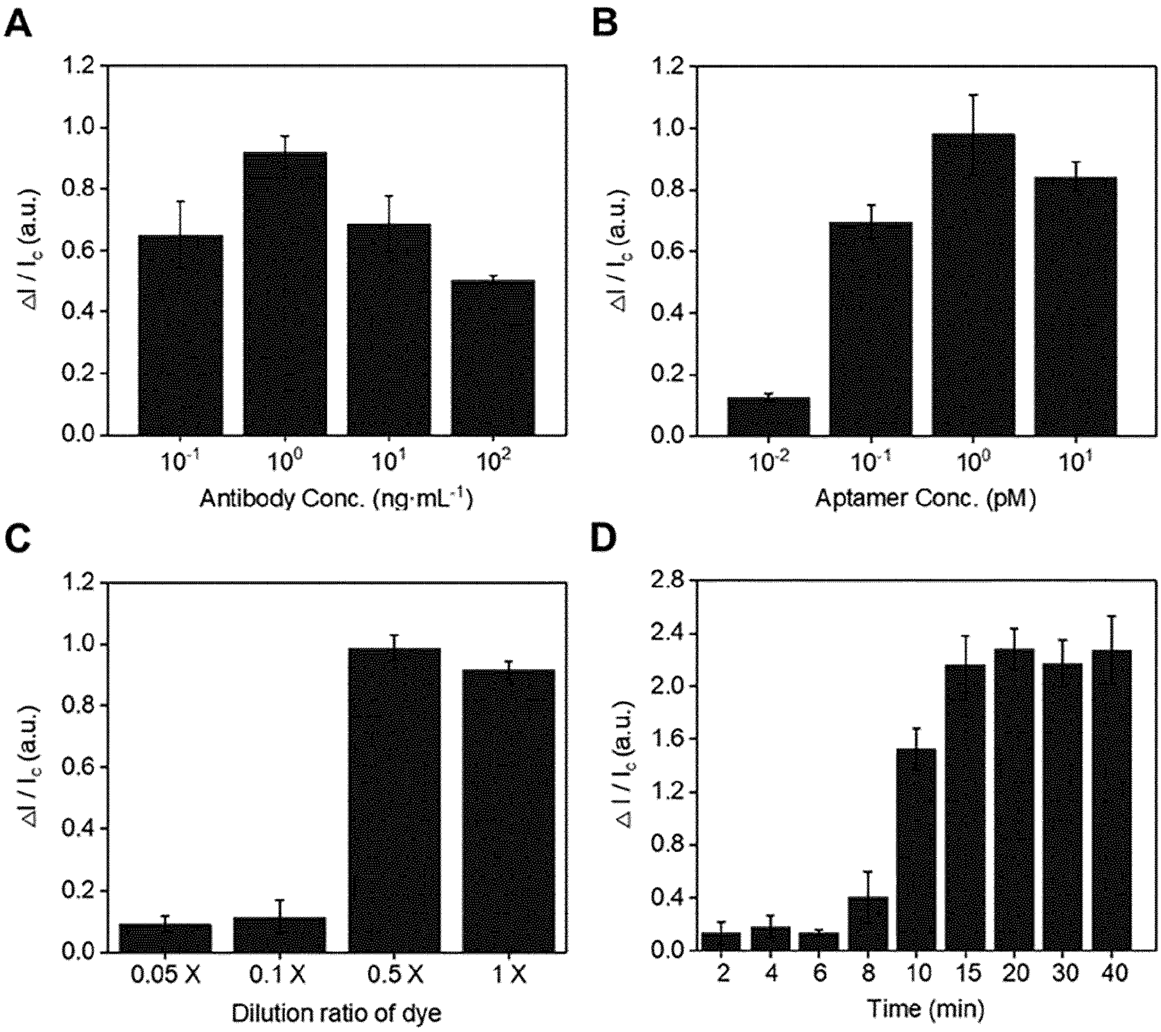
A
1.2
1.0
0.8
0.6
0.4
0.2
0.0
ΔI / Ic (a.u.)
10-1
100
101
102
Antibody Conc. (ng·mL-1)
B
ΔI / Ic (a.u.)
10-2
10-1
100
101
Aptamer Conc. (pM)
C
ΔI / Ic (a.u.)
0.05 X
0.1 X
0.5 X
1 X
Dilution ratio of dye
D
2.8
2.4
2.0
1.6
1.2
0.8
0.4
0.0
Δ I / Ic (a.u.)
2
4
6
8
10
15
20
30
40
Time (min)

[FIG. 8]

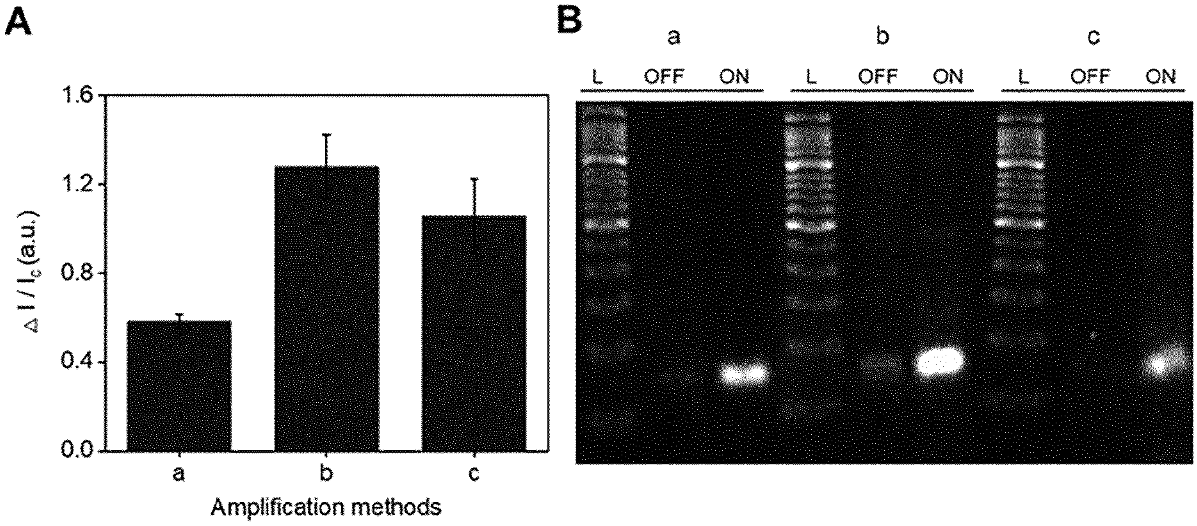
A
1.6
1.2
0.8
0.4
0.0
Δ I / I_c (a.u.)
a
b
c
Amplification methods
B
a
b
c
L OFF ON
L OFF ON
L OFF ON

[FIG. 9]

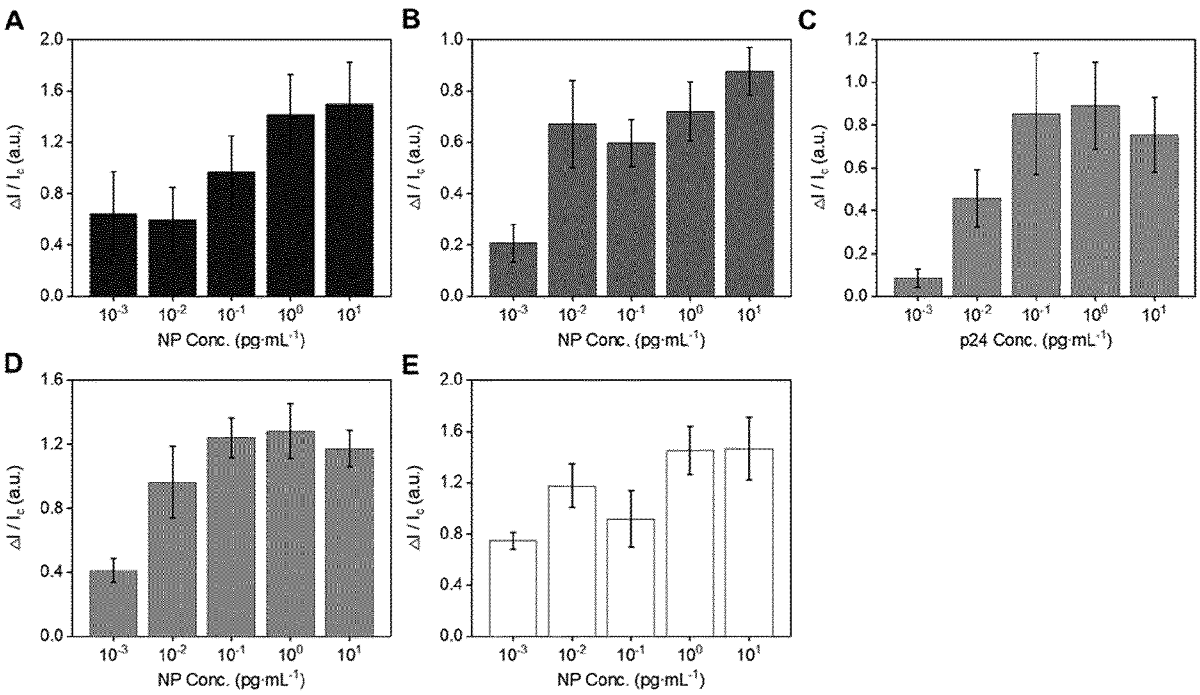
A
ΔI / I_c (a.u.)
NP Conc. (pg·mL⁻¹)
B
ΔI / I_c (a.u.)
NP Conc. (pg·mL⁻¹)
C
ΔI / I_c (a.u.)
p24 Conc. (pg·mL⁻¹)
D
ΔI / I_c (a.u.)
NP Conc. (pg·mL⁻¹)
E
ΔI / I_c (a.u.)
NP Conc. (pg·mL⁻¹)
10⁻³ 10⁻² 10⁻¹ 10⁰ 10¹

[FIG. 10]

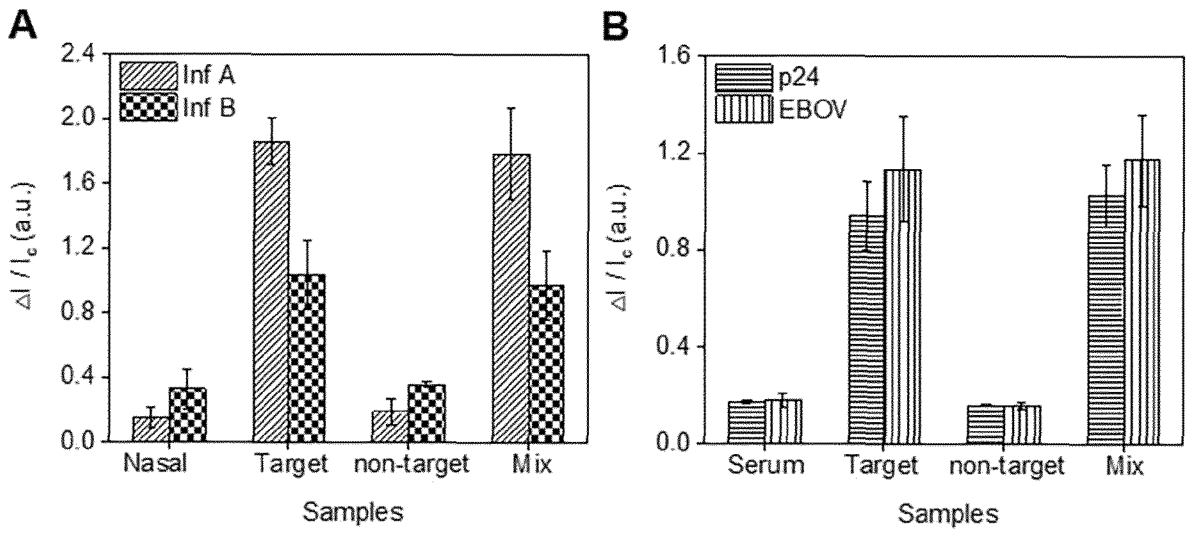
A
2.4
2.0
1.6
1.2
0.8
0.4
0.0
ΔI / I_c (a.u.)
Inf A
Inf B
Nasal
Target
non-target
Mix
Samples
B
1.6
1.2
0.8
0.4
0.0
ΔI / I_c (a.u.)
p24
EBOV
Serum
Target
non-target
Mix
Samples

**INTERNATIONAL SEARCH REPORT**

International application No.
**PCT/KR2020/008759**

**A. CLASSIFICATION OF SUBJECT MATTER**

**C12N 15/10**(2006.01)i; **C12N 15/115**(2010.01)i; **C12Q 1/68**(2006.01)i; **G01N 33/543**(2006.01)i

According to International Patent Classification (IPC) or to both national classification and IPC

**B. FIELDS SEARCHED**

Minimum documentation searched (classification system followed by classification symbols)

C12N 15/10; C12N 15/115; C12Q 1/68; G01N 33/543

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 항체(antigen), 표적(target), 압타머(aptamer), 단일가닥 DNA(single-single DNA), 면역 분석(immunoassay), 이종 샌드위치 DNA(heterogeneous sandwich DNA), 재조합효소-중합효소 증폭법(recombinase polymerase amplification)

**C. DOCUMENTS CONSIDERED TO BE RELEVANT**

| Category* | Citation of document, with indication, where appropriate, of the relevant passages | Relevant to claim No. |
|---|---|---|
| X<br>A | KANG, Juyoung et al. Development of a DNA aptamer selection method based on the heterogeneous sandwich form and its application in a colorimetric assay for influenza A virus detection. New Journal of Chemistry. 2019, vol. 43, pp. 6883-6885 (published online on 03 April 2019). See abstract, pages 6884-6885 and Fig. 1. | 1-11<br>12-23 |
| A | KR 10-2017-0021424 A (VIROMED CO., LTD.) 28 February 2017. See paragraphs [0014]-[0025]. | 1-23 |
| A | KR 10-2014-0015455 A (SOMALOGIC, INC.) 06 February 2014. See claims 24-34. | 1-23 |
| A | KR 10-2010-0044787 A (SOMALOGIC, INC.) 30 April 2010. See claims 1-17. | 1-23 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **08 October 2020** | **08 October 2020** |

| Name and mailing address of the ISA/KR | Authorized officer |
|---|---|
| **Korean Intellectual Property Office**<br>**Government Complex Daejeon Building 4, 189, Cheongsa-ro, Seo-gu, Daejeon, Republic of Korea**<br>**35208** | |
| Facsimile No. **+82-42-481-8578** | Telephone No. |

Form PCT/ISA/210 (second sheet) (July 2019)

**INTERNATIONAL SEARCH REPORT**

International application No.
**PCT/KR2020/008759**

**C. DOCUMENTS CONSIDERED TO BE RELEVANT**

| Category* | Citation of document, with indication, where appropriate, of the relevant passages | Relevant to claim No. |
|---|---|---|
| A | KR 10-2015-0030655 A (SOMALOGIC, INC.) 20 March 2015. See claims 1-32. | 1-23 |
| PX | KR 10-2113078 B1 (GWANGJU INSTITUTE OF SCIENCE AND TECHNOLOGY) 20 May 2020. See claims 1-8. *The above document is a published earlier application that serves as a basis for claiming priority of the present international application. | 1-9 |

Form PCT/ISA/210 (second sheet) (July 2019)

**INTERNATIONAL SEARCH REPORT**
**Information on patent family members**

International application No.
**PCT/KR2020/008759**

| Patent document cited in search report | Publication date (day/month/year) | Patent family member(s) | Publication date (day/month/year) |
|---|---|---|---|
| KR 10-2017-0021424 A | 28 February 2017 | None | |
| KR 10-2014-0015455 A | 06 February 2014 | AU 2012-225273 A1 | 12 September 2013 |
| | | AU 2012-225273 A1 | 13 September 2012 |
| | | AU 2012-225273 B2 | 13 October 2016 |
| | | AU 2014-326975 A1 | 02 April 2015 |
| | | AU 2014-326975 A1 | 07 April 2016 |
| | | AU 2014-326975 A2 | 02 April 2015 |
| | | AU 2014-326975 A2 | 05 January 2017 |
| | | AU 2014-326975 B2 | 07 May 2020 |
| | | AU 2016-256840 A1 | 01 December 2016 |
| | | AU 2016-256840 B2 | 02 November 2017 |
| | | BR 112016005957 A2 | 26 September 2017 |
| | | CA 2828286 A1 | 13 September 2012 |
| | | CA 2828286 C | 03 April 2018 |
| | | CA 2924987 A1 | 02 April 2015 |
| | | CN 105555957 A | 04 May 2016 |
| | | EP 2683729 A1 | 15 January 2014 |
| | | EP 2683729 B1 | 03 May 2017 |
| | | EP 3049523 A1 | 03 August 2016 |
| | | EP 3049523 B1 | 07 August 2019 |
| | | ES 2629183 T3 | 07 August 2017 |
| | | HK 1218765 A1 | 10 March 2017 |
| | | IL 244284 A | 21 April 2016 |
| | | IL 244284 D0 | 21 April 2016 |
| | | JP 2014-513929 A | 19 June 2014 |
| | | JP 2016-531593 A | 13 October 2016 |
| | | JP 6018096 B2 | 02 November 2016 |
| | | JP 6506768 B2 | 24 April 2019 |
| | | KR 10-2016-0058777 A | 25 May 2016 |
| | | MX 2016003320 A | 31 May 2016 |
| | | MX 361451 B | 06 December 2018 |
| | | SG 11201602063 A | 28 April 2016 |
| | | US 0392621 B2 | 27 August 2019 |
| | | US 2012-0231467 A1 | 13 September 2012 |
| | | US 2015-0031585 A1 | 29 January 2015 |
| | | US 2015-0125867 A1 | 07 May 2015 |
| | | US 2016-0237435 A1 | 18 August 2016 |
| | | US 2018-187197 A1 | 05 July 2018 |
| | | US 8895241 B2 | 25 November 2014 |
| | | US 9081010 B2 | 14 July 2015 |
| | | US 9926566 B2 | 27 March 2018 |
| | | WO 2012-122540 A1 | 13 September 2012 |
| | | WO 2015-048084 A1 | 02 April 2015 |
| KR 10-2010-0044787 A | 30 April 2010 | AT 530665 T | 15 November 2011 |
| | | AU 1823199 A | 05 July 1999 |
| | | AU 1999-18231 A1 | 05 July 1999 |
| | | AU 1999-18231 B2 | 28 November 2002 |
| | | AU 2003-200718 B2 | 19 October 2006 |
| | | AU 2003-217379 A1 | 09 September 2003 |
| | | AU 2007-200237 A1 | 08 February 2007 |

Form PCT/ISA/210 (patent family annex) (July 2019)

**INTERNATIONAL SEARCH REPORT**
**Information on patent family members**

International application No.
**PCT/KR2020/008759**

| Patent document cited in search report | Publication date (day/month/year) | Patent family member(s) | Publication date (day/month/year) |
|---|---|---|---|
| | | AU 2007-200237 B2 | 17 February 2011 |
| | | AU 2007-205974 A1 | 26 July 2007 |
| | | AU 2008-275915 A1 | 22 January 2009 |
| | | AU 2008-275915 A2 | 22 January 2009 |
| | | AU 2008-275915 A2 | 22 April 2010 |
| | | AU 2008-275915 B2 | 11 July 2013 |
| | | AU 2008-275917 A1 | 22 January 2009 |
| | | AU 2008-275917 A2 | 22 January 2009 |
| | | AU 2008-275917 A2 | 29 April 2010 |
| | | AU 2008-275917 B2 | 21 August 2014 |
| | | AU 2008-276001 A1 | 22 January 2009 |
| | | AU 2008-276001 A2 | 03 June 2010 |
| | | AU 2008-276001 A2 | 22 January 2009 |
| | | AU 2008-276001 A9 | 22 January 2009 |
| | | AU 2008-276001 A9 | 10 June 2010 |
| | | AU 2010-271271 A1 | 13 January 2011 |
| | | AU 2010-271271 A1 | 19 January 2012 |
| | | AU 2010-271271 B2 | 29 January 2015 |
| | | AU 2013-203360 A1 | 02 May 2013 |
| | | AU 2013-203360 B2 | 10 December 2015 |
| | | AU 2013-203374 A1 | 02 May 2013 |
| | | AU 2013-203374 B2 | 09 April 2015 |
| | | AU 2013-203386 A1 | 02 May 2013 |
| | | AU 2013-203386 B2 | 08 October 2015 |
| | | AU 2013-203588 A1 | 02 May 2013 |
| | | AU 2013-203588 B2 | 29 October 2015 |
| | | AU 2013-203588 C1 | 30 June 2016 |
| | | AU 2013-248169 A1 | 30 January 2014 |
| | | AU 2013-248169 B2 | 10 December 2015 |
| | | AU 2015-249081 A1 | 12 November 2015 |
| | | AU 2015-249081 B2 | 02 March 2017 |
| | | AU 2015-249082 A1 | 12 November 2015 |
| | | AU 2015-249082 B2 | 02 March 2017 |
| | | AU 2015-255156 A1 | 26 November 2015 |
| | | AU 2015-255156 B2 | 19 January 2017 |
| | | AU 2015-255156 C1 | 11 May 2017 |
| | | AU 2015-261546 A1 | 10 December 2015 |
| | | AU 2015-261546 B2 | 28 September 2017 |
| | | AU 2017-202051 A1 | 20 April 2017 |
| | | AU 2017-202051 B2 | 31 January 2019 |
| | | AU 2017-202493 A1 | 04 May 2017 |
| | | AU 2017-202493 B2 | 28 February 2019 |
| | | AU 2019-201066 A1 | 07 March 2019 |
| | | AU 754956 B2 | 28 November 2002 |
| | | CA 2312429 A1 | 24 June 1999 |
| | | CA 2312429 C | 08 December 2015 |
| | | CA 2476309 A1 | 28 August 2003 |
| | | CA 2634987 A1 | 26 July 2007 |
| | | CA 2634987 C | 03 January 2017 |
| | | CA 2693448 A1 | 22 January 2009 |

Form PCT/ISA/210 (patent family annex) (July 2019)

**INTERNATIONAL SEARCH REPORT**
**Information on patent family members**

International application No.
**PCT/KR2020/008759**

| Patent document cited in search report | Publication date (day/month/year) | Patent family member(s) | Publication date (day/month/year) |
|---|---|---|---|
| | | CA 2693453 A1 | 22 January 2009 |
| | | CA 2693453 C | 11 December 2018 |
| | | CA 2696431 A1 | 22 January 2009 |
| | | CA 2765857 A1 | 13 January 2011 |
| | | CA 2765857 C | 06 March 2018 |
| | | CA 2982304 A1 | 13 January 2011 |
| | | CA 3022666 A1 | 22 January 2009 |
| | | CN 101374965 A | 25 February 2009 |
| | | CN 101374965 B | 14 August 2013 |
| | | CN 101802225 A | 11 August 2010 |
| | | CN 101802225 B | 30 October 2013 |
| | | CN 101809167 A | 18 August 2010 |
| | | CN 101809167 B | 01 April 2015 |
| | | CN 103627792 A | 12 March 2014 |
| | | CN 104593373 A | 06 May 2015 |
| | | CN 107090457 A | 25 August 2017 |
| | | DK 1994171 T3 | 15 June 2015 |
| | | DK 2069529 T3 | 15 April 2013 |
| | | DK 2172566 T3 | 04 May 2015 |
| | | DK 2280083 T3 | 30 September 2013 |
| | | DK 2489743 T3 | 02 March 2015 |
| | | EP 1040203 A1 | 04 October 2000 |
| | | EP 1040203 B1 | 26 October 2011 |
| | | EP 1481090 A1 | 01 December 2004 |
| | | EP 1994171 A2 | 26 November 2008 |
| | | EP 1994171 B1 | 11 March 2015 |
| | | EP 2069496 A1 | 17 June 2009 |
| | | EP 2069529 A2 | 17 June 2009 |
| | | EP 2069529 B1 | 09 January 2013 |
| | | EP 2076613 A1 | 08 July 2009 |
| | | EP 2076613 B1 | 07 September 2016 |
| | | EP 2172566 A1 | 07 April 2010 |
| | | EP 2172566 B1 | 04 March 2015 |
| | | EP 2280083 A1 | 02 February 2011 |
| | | EP 2280083 B1 | 19 June 2013 |
| | | EP 2336314 A1 | 22 June 2011 |
| | | EP 2451981 A1 | 16 May 2012 |
| | | EP 2451981 B1 | 19 October 2016 |
| | | EP 2489743 A2 | 22 August 2012 |
| | | EP 2489743 A3 | 21 November 2012 |
| | | EP 2489743 B1 | 07 January 2015 |
| | | EP 2613147 A1 | 10 July 2013 |
| | | EP 2613147 B1 | 11 March 2015 |
| | | EP 2626436 A2 | 14 August 2013 |
| | | EP 2626436 A3 | 16 October 2013 |
| | | EP 2933340 A1 | 21 October 2015 |
| | | EP 2933340 B1 | 06 September 2017 |
| | | EP 2952894 A1 | 09 December 2015 |
| | | EP 2952894 B1 | 30 August 2017 |
| | | EP 3284832 A2 | 21 February 2018 |

Form PCT/ISA/210 (patent family annex) (July 2019)

**INTERNATIONAL SEARCH REPORT**
**Information on patent family members**

International application No.
**PCT/KR2020/008759**

| Patent document cited in search report | Publication date (day/month/year) | Patent family member(s) | Publication date (day/month/year) |
|---|---|---|---|
| | | EP 3284832 A3 | 07 March 2018 |
| | | ES 2533711 T3 | 14 April 2015 |
| | | ES 2537322 T3 | 05 June 2015 |
| | | ES 2538038 T3 | 16 June 2015 |
| | | ES 2538121 T3 | 17 June 2015 |
| | | ES 2604764 T3 | 09 March 2017 |
| | | ES 2610633 T3 | 28 April 2017 |
| | | ES 2644499 T3 | 29 November 2017 |
| | | ES 2647587 T3 | 22 December 2017 |
| | | HK 1031401 A1 | 10 February 2012 |
| | | HK 1127093 A1 | 25 April 2014 |
| | | HK 1132012 A1 | 28 July 2017 |
| | | HK 1133046 A1 | 07 June 2013 |
| | | HK 1141839 A1 | 25 September 2015 |
| | | HK 1174366 A1 | 11 September 2015 |
| | | HK 1213020 A1 | 24 June 2016 |
| | | HK 1217540 A1 | 13 January 2017 |
| | | HK 1246368 A1 | 07 September 2018 |
| | | HR P20150382 T1 | 08 May 2015 |
| | | HU E025489 T2 | 28 April 2016 |
| | | JP 2002-508191 A | 19 March 2002 |
| | | JP 2005-517456 A | 16 June 2005 |
| | | JP 2009-159965 A | 23 July 2009 |
| | | JP 2009-523430 A | 25 June 2009 |
| | | JP 2010-533499 A | 28 October 2010 |
| | | JP 2010-533872 A | 28 October 2010 |
| | | JP 2012-532612 A | 20 December 2012 |
| | | JP 2014-050398 A | 20 March 2014 |
| | | JP 2015-027301 A | 12 February 2015 |
| | | JP 2015-062416 A | 09 April 2015 |
| | | JP 2016-065872 A | 28 April 2016 |
| | | JP 2016-122014 A | 07 July 2016 |
| | | JP 2016-171810 A | 29 September 2016 |
| | | JP 2017-104122 A | 15 June 2017 |
| | | JP 2019-004901 A | 17 January 2019 |
| | | JP 2019-039929 A | 14 March 2019 |
| | | JP 2020-078311 A | 28 May 2020 |
| | | JP 5404620 B2 | 05 February 2014 |
| | | JP 5680827 B2 | 04 March 2015 |
| | | JP 5684568 B2 | 11 March 2015 |
| | | JP 5926178 B2 | 25 May 2016 |
| | | JP 5938080 B2 | 22 June 2016 |
| | | JP 5956404 B2 | 27 July 2016 |
| | | JP 6144654 B2 | 07 June 2017 |
| | | JP 6407195 B2 | 17 October 2018 |
| | | JP 6491610 B2 | 27 March 2019 |
| | | KR 10-0603115 B1 | 20 July 2006 |
| | | KR 10-1149688 B1 | 24 May 2012 |
| | | KR 10-1634608 B1 | 29 June 2016 |
| | | KR 10-1656240 B1 | 09 September 2016 |

Form PCT/ISA/210 (patent family annex) (July 2019)

**INTERNATIONAL SEARCH REPORT**
**Information on patent family members**

International application No.
**PCT/KR2020/008759**

| Patent document cited in search report | Publication date (day/month/year) | Patent family member(s) | Publication date (day/month/year) |
|---|---|---|---|
| | | KR 10-1747665 B1 | 15 June 2017 |
| | | KR 10-2001-0033184 A | 25 April 2001 |
| | | KR 10-2008-0104116 A | 01 December 2008 |
| | | KR 10-2010-0058485 A | 03 June 2010 |
| | | KR 10-2016-0108584 A | 19 September 2016 |
| | | MX 2010000533 A | 30 April 2010 |
| | | MX 2010000578 A | 30 April 2010 |
| | | MX 341491 B | 23 August 2016 |
| | | MX 344253 B | 09 December 2016 |
| | | MX 355017 B | 02 April 2018 |
| | | NO 2933340 T3 | 03 February 2018 |
| | | PL 1994171 T3 | 31 August 2015 |
| | | PL 2489743 T3 | 30 June 2015 |
| | | PT 1994171 E | 06 July 2015 |
| | | PT 2172566 E | 23 June 2015 |
| | | PT 2489743 E | 09 April 2015 |
| | | SI 1994171 T1 | 31 July 2015 |
| | | SI 2489743 T1 | 30 April 2015 |
| | | SI EP1994171 T1 | 31 July 2015 |
| | | SI EP2489743 T1 | 30 April 2015 |
| | | TW 200801203 A | 01 January 2008 |
| | | TW I507528 B | 11 November 2015 |
| | | US 0316321 B2 | 11 June 2019 |
| | | US 0422794 B2 | 24 September 2019 |
| | | US 0648972 B2 | 12 May 2020 |
| | | US 2003-0162216 A1 | 28 August 2003 |
| | | US 2006-0057573 A1 | 16 March 2006 |
| | | US 2007-0166740 A1 | 19 July 2007 |
| | | US 2007-0166741 A1 | 19 July 2007 |
| | | US 2007-0166742 A1 | 19 July 2007 |
| | | US 2008-0160535 A1 | 03 July 2008 |
| | | US 2009-0004667 A1 | 01 January 2009 |
| | | US 2009-0042206 A1 | 12 February 2009 |
| | | US 2009-098549 A1 | 16 April 2009 |
| | | US 2010-055695 A1 | 04 March 2010 |
| | | US 2010-209935 A1 | 19 August 2010 |
| | | US 2010-317120 A1 | 16 December 2010 |
| | | US 2011-082286 A1 | 07 April 2011 |
| | | US 2011-136099 A1 | 09 June 2011 |
| | | US 2011-245479 A1 | 06 October 2011 |
| | | US 2012-115752 A1 | 10 May 2012 |
| | | US 2012-264117 A1 | 18 October 2012 |
| | | US 2014-249043 A1 | 04 September 2014 |
| | | US 2014-296102 A1 | 02 October 2014 |
| | | US 2015-148237 A1 | 28 May 2015 |
| | | US 2015-168388 A1 | 18 June 2015 |
| | | US 2015-197753 A1 | 16 July 2015 |
| | | US 2016-265038 A1 | 15 September 2016 |
| | | US 2017-137819 A1 | 18 May 2017 |
| | | US 2018-155725 A1 | 07 June 2018 |

Form PCT/ISA/210 (patent family annex) (July 2019)

**INTERNATIONAL SEARCH REPORT**
**Information on patent family members**

International application No.
**PCT/KR2020/008759**

| Patent document cited in search report | Publication date (day/month/year) | Patent family member(s) | Publication date (day/month/year) |
|---|---|---|---|
| | | US 2018-156787 A1 | 07 June 2018 |
| | | US 2019-264208 A1 | 29 August 2019 |
| | | US 2019-330634 A1 | 31 October 2019 |
| | | US 6242246 B1 | 05 June 2001 |
| | | US 6458543 B1 | 01 October 2002 |
| | | US 6503715 B1 | 07 January 2003 |
| | | US 6544776 B1 | 08 April 2003 |
| | | US 7709192 B2 | 04 May 2010 |
| | | US 7855054 B2 | 21 December 2010 |
| | | US 7947447 B2 | 24 May 2011 |
| | | US 7964356 B2 | 21 June 2011 |
| | | US 8071288 B2 | 06 December 2011 |
| | | US 8404830 B2 | 26 March 2013 |
| | | US 8409795 B2 | 02 April 2013 |
| | | US 8703416 B2 | 22 April 2014 |
| | | US 8945830 B2 | 03 February 2015 |
| | | US 8975026 B2 | 10 March 2015 |
| | | US 8975388 B2 | 10 March 2015 |
| | | US 9382533 B2 | 05 July 2016 |
| | | US 9404919 B2 | 02 August 2016 |
| KR 10-2015-0030655 A | 20 March 2015 | AU 2013-271401 A1 | 27 November 2014 |
| | | AU 2013-271401 A1 | 12 December 2013 |
| | | AU 2013-271401 B2 | 05 April 2018 |
| | | BR 112014030298 A2 | 27 June 2017 |
| | | CA 2873099 A1 | 12 December 2013 |
| | | CN 104508150 A | 08 April 2015 |
| | | CN 108614102 A | 02 October 2018 |
| | | CY 1118748 T1 | 12 July 2017 |
| | | EP 2859121 A1 | 15 April 2015 |
| | | EP 2859121 B1 | 30 November 2016 |
| | | ES 2617226 T3 | 15 June 2017 |
| | | ES 2617226 T8 | 27 August 2019 |
| | | HK 1204341 A1 | 13 November 2015 |
| | | HR P20170296 T1 | 21 April 2017 |
| | | IL 235588 A | 29 January 2015 |
| | | IL 235588 B | 31 July 2018 |
| | | IL 235588 D0 | 29 January 2015 |
| | | IN 2647KON2014 A | 08 May 2015 |
| | | JP 2015-520385 A | 16 July 2015 |
| | | JP 6298456 B2 | 20 March 2018 |
| | | KR 10-2088381 B1 | 13 March 2020 |
| | | MX 2014014784 A | 19 March 2015 |
| | | MX 356413 B | 29 May 2018 |
| | | NZ 701824 A | 27 January 2017 |
| | | PH 12014502581 A1 | 21 January 2015 |
| | | PH 12014502581 B1 | 21 January 2015 |
| | | RU 2014152698 A | 10 August 2016 |
| | | RU 2666989 C2 | 13 September 2018 |
| | | SG 10201701361 A | 27 April 2017 |
| | | SG 11201407505 A | 30 December 2014 |

Form PCT/ISA/210 (patent family annex) (July 2019)

**INTERNATIONAL SEARCH REPORT**
**Information on patent family members**

International application No.
**PCT/KR2020/008759**

| Patent document cited in search report | Publication date (day/month/year) | Patent family member(s) | Publication date (day/month/year) |
|---|---|---|---|
| | | US 2015-0141259 A1 | 21 May 2015 |
| | | US 2017-176422 A1 | 22 June 2017 |
| | | US 2018-224444 A1 | 09 August 2018 |
| | | WO 2013-185078 A1 | 12 December 2013 |
| | | ZA 201408409 B | 30 August 2017 |
| KR 10-2113078 B1 | 20 May 2020 | None | |

Form PCT/ISA/210 (patent family annex) (July 2019)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 53642890
- US 5475096 A
- US 5270163 A
- US 5789163 A


### Non-patent literature cited in the description


- **FISCHER, NICHOLAS O et al.** Protein detection via direct enzymatic amplification of short DNA aptamers. *Analytical biochemistry,* 2008, vol. 373 (1), 121-8

<110>    Gwangju Institute of Science and Technology

<120>    A SCREENING METHOD OF APTAMER AND IMMUNOASSAY USING THE APTAMER

<130>    Gpo-00042PCT

<160>    10

<170>    KoPatentIn 3.0

<210>    1
<211>    245
<212>    PRT
<213>    Artificial Sequence

<220>
<223>    Severe fever with thrombocytopenia virus N protein

<400>    1
```
Met Ser Glu Trp Ser Arg Ile Ala Val Glu Phe Gly Glu Gln Gln Leu
  1               5                  10                  15

Asn Leu Thr Glu Leu Glu Asp Phe Ala Arg Glu Leu Ala Tyr Glu Gly
             20                  25                  30

Leu Asp Pro Ala Leu Ile Ile Lys Lys Leu Lys Glu Thr Gly Gly Asp
         35                  40                  45

Asp Trp Val Lys Asp Thr Lys Phe Ile Ile Val Phe Ala Leu Thr Arg
     50                  55                  60

Gly Asn Lys Ile Val Lys Ala Ser Gly Lys Met Ser Asn Ser Gly Ser
 65                  70                  75                  80

Lys Arg Leu Met Ala Leu Gln Glu Lys Tyr Gly Leu Val Glu Arg Ala
                 85                  90                  95

Glu Thr Arg Leu Ser Ile Thr Pro Val Arg Val Ala Gln Ser Leu Pro
            100                 105                 110

Thr Trp Thr Cys Ala Ala Ala Ala Ala Leu Lys Glu Tyr Leu Pro Val
        115                 120                 125

Gly Pro Ala Val Met Asn Leu Lys Val Glu Asn Tyr Pro Pro Glu Met
    130                 135                 140

Met Cys Met Ala Phe Gly Ser Leu Ile Pro Thr Ala Gly Val Ser Glu
145                 150                 155                 160

Ala Thr Thr Lys Thr Leu Met Glu Ala Tyr Ser Leu Trp Gln Asp Ala
                165                 170                 175

Phe Thr Lys Thr Ile Asn Val Lys Met Arg Gly Ala Ser Lys Thr Glu
            180                 185                 190

Val Tyr Asn Ser Phe Arg Asp Pro Leu His Ala Ala Val Asn Ser Val
        195                 200                 205

Phe Phe Pro Asn Asp Val Arg Val Lys Trp Leu Lys Ala Lys Gly Ile
    210                 215                 220

Leu Gly Pro Asp Gly Val Pro Ser Arg Ala Ala Glu Val Ala Ala Ala
```

225 230 235 240

Ala Tyr Arg Asn Leu
245

<210> 2
<211> 76
<212> DNA
<213> Artificial Sequence

<220>
<223> Synthetic (Nucleic acid construct)

<220>
<221> misc_feature
<222> (19)..(58)
<223> each n is A, T, U, G or C

<400> 2
atccagagtg acgcagcann nnnnnnnnnn nnnnnnnnnn nnnnnnnnnn nnnnnnnntg 60

gacacggtgg cttagt 76

<210> 3
<211> 40
<212> DNA
<213> Artificial Sequence

<220>
<223> aptamer core sequence

<400> 3
cgaccacaga ttggagactg atagtgcacg agcaaggaca 40

<210> 4
<211> 40
<212> DNA
<213> Artificial Sequence

<220>
<223> aptamer core sequence

<400> 4
tcggatggat tgtggtcgaa gttgtttccg acactagtca 40

<210> 5
<211> 40
<212> DNA
<213> Artificial Sequence

<220>
<223> aptamer core sequence

<400> 5

cacatcggag aacaggcgca ctgtcggagg aaccgcaacg 40

<210> 6
<211> 76
<212> DNA
<213> Artificial Sequence

<220>
<223> Influenza A NP aptamer

<400> 6
tagggaagag aaggacatat gatggcgtac ggggatgagg tgatcgtagt gggttgacta 60

gtacatgacc acttga 76

<210> 7
<211> 80
<212> DNA
<213> Artificial Sequence

<220>
<223> Influenza B NP aptamer

<400> 7
attatggcgt ttgcagcgtt ctggttggtg gtggtgatag gtgggggaa ggagggtatc 60

ttgttggtga ggtaacggct 80

<210> 8
<211> 82
<212> DNA
<213> Artificial Sequence

<220>
<223> human immunodeficiency virus (HIV) p24 aptamer

<400> 8
agatactgcc attcattgca tcgagcacgc gactgatgag gatggtctag tagctggggt 60

cgagtactaa gctatgtgtc ga 82

<210> 9
<211> 85
<212> DNA
<213> Artificial Sequence

<220>
<223> Ebola NP aptamer

<400> 9
gatgtgagtg acgtggatcg agcggatgtg aaggctgaaa gtggctttgg gcggtcgtaa 60

gtgtcacaga gcatgcaaca agacc 85

<210> 10
<211> 76
<212> DNA
<213> Artificial Sequence

<220>
<223> SARS-CoV-2 NP aptamer

<400> 10
atccagagtg acgcagcaaa cccaagcaaa ctacctctat acccttcgac cttcatcatg 60

gacacggtgg cttagt 76